(19)

**Europäisches Patentamt**
**European Patent Office**
**Office européen des brevets**

(11) **EP 3 789 045 A1**

(12) **EUROPÄISCHE PATENTANMELDUNG**

(43) Veröffentlichungstag:
**10.03.2021 Patentblatt 2021/10**

(21) Anmeldenummer: **19195459.3**

(22) Anmeldetag: **04.09.2019**

(51) Int Cl.:
*A61L 2/18* (2006.01)     *B01F 15/04* (2006.01)
*B65D 25/08* (2006.01)     *B65D 81/32* (2006.01)
*A61L 2/14* (2006.01)     *A01N 59/00* (2006.01)
*A23L 3/358* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Leibniz-Institut für Plasmaforschung und Technologie e.V.**
**17489 Greifswald (DE)**

(72) Erfinder:
• **SCHMIDT-BLEKER, Dr. Ansgar**
**33729 Bielefeld (DE)**
• **WINTER, Dr. Jörn**
**17489 Greifswald (DE)**
• **WELTMANN, Klaus-Dieter**
**18609 Ostseebad Binz (DE)**

(74) Vertreter: **Schulz Junghans Patentanwälte PartGmbB Großbeerenstraße 71 10963 Berlin (DE)**

(54) **DESINFEKTIONSVERFAHREN MIT EINEM DURCH REAKTION VON H2O2 UND NO2 IN SITU GEBILDETEM DESINFEKTIONSWIRKSTOFF MIT RETARDIERTER WIRKSTOFFFREISETZUNG**

(57) Die Erfindung betrifft ein Verfahren zur Desinfektion von Oberflächen umfassend die Bereitstellung einer Wirklösung umfassend die Edukte $H_2O_2$ und $NO_2^-$, wobei die Wirklösung mindestens ein Stoppagens umfasst, wobei das Stoppagens ein Lösungsmittel ist, welches eine Siedetemperatur unter 100 °C aufweist. Weiterhin betrifft die Erfindung eine Vorrichtung zur Anwendung dieses Verfahren.

Fig. 14

**EP 3 789 045 A1**

**Beschreibung**

Hintergrund der Erfindung

Die vorliegende Erfindung betrifft ein Verfahren und eine Vorrichtung zur Desinfektion von Oberflächen, insbesondere zur Desinfektion von Körperteilen, insbesondere von Händen, und/oder insbesondere zur Desinfektion von Wunden.

Die biozide Wirkung der Reaktionsprodukte von Wasserstoffperoxid ($H_2O_2$) und Nitrit ($NO_2^-$) unter Zugabe einer Säure ist bereits in der Literatur bekannt.

Beispielsweise kann durch das Vermischen zweier Ausgangslösungen, von denen eine Ausgangslösung $H_2O_2$ und eine $NO_2^-$ haltig ist, eine desinfizierende Wirkung erreicht werden, sofern die Ungleichung

$$W = \int_{t_1}^{t_2} k \cdot [H_2O_2] \cdot [NO_2^-]\, dt \geq W_{\min} \qquad (1)$$

erfüllt ist. Hierin ist $W$ der sogenannte Wirksamkeitsparameter, der größer als $W_{\min}$ sein muss, um eine Wirkung zu erhalten. Der Wert $W_{\min}$ kann vom jeweils zu inaktivierenden Mikroorganismus abhängen. Ferner ist $k$ die Reaktionsrate der Reaktion

$$H_2O_2 + NO_2^- \rightarrow \text{Reaktionsprodukte}, \qquad (2)$$

welche unter Anderem zur Bildung von kurzlebigen reaktiven Spezies, insbesondere von Peroxinitritsäure führt. Ferner berücksichtigt (1), dass bei der Oberflächendekontamination ein Verteilschritt durchzuführen ist. Dieser beginnt, nachdem die Flüssigkeiten vermischt wurden, am Zeitpunkt $t_0$ und endet zum Zeitpunkt $t_1 > t_0$. Hierbei bezeichnet $t_1$ den Zeitpunkt, bei welchem die zu desinfizierende Oberfläche vollständig benetzt ist. Die Einwirkzeit selbst startet demzufolge erst zum Zeitpunkt $t_1$ und endet zum Zeitpunkt $t_2$.

Bekannte Verfahren haben für die Oberflächendekontamination den Nachteil, dass die Reaktionsrate

$$R = k \cdot [H_2O_2] \cdot [NO_2^-], \qquad (3)$$

welche nach Gleichung (1) für die biozide Wirkung verantwortlich ist, direkt nach Vermischen der beiden Ausgangslösungen am höchsten ist, da dann nach Gleichung (2) auch die Konzentration der Edukte am größten ist. Die Reaktionsrate ist für $t > t_0$ streng monoton fallend. Da der Einwirkzeitraum erst zum Zeitpunkt $t_1 > t_0$ beginnt, hat dies zur Folge, dass vor dem eigentlichen Desinfektionsprozess schon ein Teil der Edukte miteinander reagiert hat und im Einwirkzeitraum nicht mehr zur Verfügung steht. Um dennoch eine ausreichende Wirkung während des Einwirkzeitraums zu gewährleisten, müssen die initialen EduktKonzentrationen zum Zeitpunkt $t_0$ deutlich höher gewählt werden. Dies ist nachteilig, da hohe

Eduktkonzentrationen erhöhte Kosten für die Desinfektionsmittelbereitstellung bedeuten und zudem durch ungewollte biozide Wirkungen mit schädlichen Folgen ein erhöhtes Risiko für die Desinfektionsanwendung während des Verteilzeitraums darstellen.

Daher zielt das im Folgenden dargestellte Verfahren auf die Dekontamination von Oberflächen mittels Mischungen aus $NO_2^-$ und $H_2O_2$ ab, wobei die Reaktion (1) durch Hinzufügen eines geeigneten Lösungsmittels retardiert wird, sodass eine gegenüber dem Stand der Technik eine wesentlich bessere Wirkung erzielt werden kann. Die Retardierung wird hierbei dadurch erreicht, dass das retardierende Lösungsmittel die Reaktionsgeschwindigkeit der Reaktion (2) verringert, wobei die retardierende Wirkung nachlässt, sobald sich die Konzentration des retardierenden Lösungsmittels in der Mischung beispielsweise durch Verdampfen des Lösungsmittels reduziert. Zudem erlaubt das erfindungsgemäße Verfahren verglichen mit bekannten Verfahren wesentlich längere Verarbeitungszeiten.

Zur gleichzeitigen Ausbringung von Fluiden sind zum Beispiel Doppelspritzen bekannt, bei denen in zwei Zylindern zwei Kolben angeordnet sind, die mechanisch miteinander außerhalb der Zylinder an einem gemeinsamen Druckelement verbunden sind, sodass bei Einleitung einer Druckkraft in das Druckelement die beiden Kolben gleichzeitig verschoben werden können und Fluide aus den Zylindern ausbringen können. Diese Konstruktion benötigt allerdings auch entsprechend lange Kolben, die vor Ausbringung der Fluide aus den Zylindern herausragen und einen entsprechenden Bauraumbedarf haben.

Dieser Aspekt ist durch eine Vorrichtung, welche zur Durchführung des erfindungsgemäßen Verfahrens geeignet ist, bereitgestellt.

Beschreibung der Erfindung

*Definitionen*

Gemäß der vorliegenden Erfindung umfasst die *Wirklösung* eine Desinfektionslösung, die auf der zu desinfizierenden Oberfläche angewendet wird. Hierbei bezeichnet die Oberfläche eine plane Fläche oder eine Fläche mit Unebenheiten und/oder Hohlräumen. Die Wirklösung kann, zusätzlich zu den in situ gebildeten desinfizierenden Wirkstoffen, noch Zusatzstoffe enthalten. Solche Zusatzstoffe umfassen unter anderem, aber nicht ausschließlich, Lösungsmittel, Pufferlösungen, Basen, Duftstoffe, Rostschutzmittel, Komplexbildner, Farbstoffe und/oder weitere Desinfektionsmittel und/oder Ozon, sowie weitere Reaktionsprodukte und reaktive Zwischenstufen der Reaktion zwischen $H_2O_2$ und $NO_2^-$. Gemäß der vorliegenden Erfindung umfasst ein *Verdünnungsschritt* die Verdünnung der Edukte mit Lösungsmitteln und/oder Zusatzstoffen. Der Verdünnungsschritt ist dem Vermischungsschritt vorangestellt oder erfolgt zeitgleich mit dem Vermischungsschritt.

Gemäß der vorliegenden Erfindung umfasst ein *Vermischungsschritt* die Vermischung von Edukten zum Erhalt der Wirklösung. Während des Vermischungsschrittes können zusätzlich auch Zusatzstoffe zu den Edukten gemischt werden. Der Vermischungsschritt kann aus mehreren Teilschritten zusammengesetzt sein. Der Vermischungsschritt beginnt zum Zeitpunkt $t_0=0$.

Gemäß der vorliegenden Erfindung umfasst ein *Verteilungsschritt* die Verteilung der Wirklösung auf der zu desinfizierenden Oberfläche. Hierbei wird jeder Punkt auf der zu desinfizierenden Oberfläche mit Wirklösung benetzt. Der Verteilungsschritt kann gleichzeitig mit dem Vermischungsschritt zum Zeitpunkt $t_0$ beginnen oder dem nachgestellt sein.

Gemäß der vorliegenden Erfindung umfasst der *Verarbeitungszeitraum $Z_A$* den Vermischungs- und den Verteilungsschritt, also den Zeitraum, der benötigt wird, um die Edukte zum Erhalt der Wirklösung zu vermischen und jeden zu desinfizierenden Punkt auf der zu desinfizierenden Oberfläche mit Wirklösung zu benetzen. Der Verarbeitungszeitraum beginnt zum Zeitpunkt $t_0=0$, an dem die Edukte zuerst miteinander in Kontakt gebracht werden und endet zum Zeitpunkt $t_1$, an dem jeder Punkt auf der zu desinfizierenden Oberfläche mit Wirklösung benetzt ist. Der pH-Wert und die Temperatur können sich innerhalb des Verarbeitungszeitraums verändern insbesondere wenn die Vermischung vor dem ersten Kontakt mit der Oberfläche erfolgt und die Oberfläche den pH-Wert und/oder die Temperatur beeinflusst. Aus diesem Grund sind pH-Wert und Temperatur während des Prozesses zeitabhängig.

Im Rahmen der Erfindung können auch vor dem Verarbeitungszeitraum, also vor dem Zeitpunkt $t_0=0$, für das Desinfektionsverfahren relevante Schritte, wie z.B. ein Verdünnungsschritt, ablaufen.

Gemäß der vorliegenden Erfindung umfasst ein *Einwirkschritt* die Einwirkung der Wirklösung auf der mit Wirklösung benetzten Oberfläche zur Desinfektion. Der Einwirkschritt wird durch den Einwirkzeitraum $Z_E$ beschrieben.

Gemäß der vorliegenden Erfindung umfasst der *Einwirkzeitraum $Z_E$* den Zeitraum, der benötigt wird, um durch die Wirklösung eine ausreichende Desinfektionswirkung zu erzielen. Der Einwirkzeitraum beginnt zum Zeitpunkt $t_1$, an dem jeder Punkt der zu desinfizierenden Oberfläche mit Wirklösung benetzt ist, und endet zum Zeitpunkt $t_2$, an dem jeder Punkt der mit Wirklösung benetzten Oberfläche desinfiziert ist.

Gemäß der vorliegenden Erfindung ist $NO_2^-$ ein Nitritsalz mit der allgemeinen Formel $M_xNO_2$, wobei M ein Alkali- oder Erdalkalimetall ist und $x=1$ oder $x=2$ sein kann. Insbesondere ist M Natrium oder Kalium und $x=1$. Das Nitritsalz kann dabei als Salz in Lösung oder als Feststoff vorliegen. Hierbei liegt $NO_2^-$ in Lösung je nach pH-Wert als Anion $NO_2^-$ oder als Säure $HNO_2$ vor.

Gemäß der vorliegenden Erfindung werden die Wirkstoffe, die die Reaktionsprodukte der Reaktion von $H_2O_2$ und $NO_2^-$, sind, in situ gebildet. In situ heißt, dass die Wirkstoffe erst bei Bedarf generiert werden.

Gemäß der vorliegenden Erfindung ist ein *Stoppagens* ein Lösungsmittel, welches eine Siedetemperatur unter 100 °C aufweist und welches die Reaktionsrate der Reaktion zwischen $H_2O_2$ und $NO_2^-$ verlangsamt.

*Beschreibung*

Ein erster Aspekt der Erfindung richtet sich auf ein Verfahren zur Desinfektion von Oberflächen umfassend die Bereitstellung einer Wirklösung umfassend die Edukte $H_2O_2$ und $NO_2^-$. Das Verfahren ist dadurch charakterisiert, dass die Wirklösung mindestens ein Stoppagens umfasst, wobei das Stoppagens ein Lösungsmittel ist, welches eine Siedetemperatur unter 100 °C aufweist.

Nach dem Vermischen der Edukte $H_2O_2$ und $NO_2^-$ zu einer Wirklösung bilden sie kurzlebige, reaktive Spezies, insbesondere Peroxinitritsäure, welche für die biozide Wirkung der Wirklösung verantwortlich sind. Wie oben beschrieben, ist die Reaktionsrate direkt nach dem Vermischen am höchsten, sodass bereits während des Mischvorgangs und anschließenden Verteilens der Wirklösung auf einer zu desinfizierenden Oberfläche ein Teil der Edukte miteinander reagiert und somit im Einwirkzeitraum nicht mehr zur Verfügung steht. Das Stoppagens verringert die Reaktionsrate der Reaktion zwischen $H_2O_2$ und $NO_2^-$. Die retardierende Wirkung lässt nach, sobald sich die Konzentration des retardierenden Lösungsmittels in der Mischung beispielsweise durch Verdampfen des Lösungsmittels reduziert. Dadurch erlaubt das erfindungsgemäße Verfahren längere Verarbeitungszeiten.

In einigen Ausführungsformen ist das Stoppagens ausgewählt ist aus einem Alkohol, einem Keton und einem Ester.

In einigen Ausführungsformen ist das Stoppagens ausgewählt aus Methanol, Ethanol, Isopropanol, Aceton, Ethylacetat und n-Propanol.

In einigen Ausführungsformen ist das Stoppagens ausgewählt aus Ethanol, Isopropanol und Aceton.

In einigen Ausführungsformen wird die Wirklösung durch das Vermischen der Edukte $H_2O_2$ und $NO_2^-$ und dem Stoppagens zum Zeitpunkt $t_0$ erhalten.

Vor dem Vermischen der Edukte können diese getrennt oder teilweise vermischt vorliegen. Beispielsweise können eine $H_2O_2$-Lösung, eine $NO_2^-$-Lösung und das Lösungsmittel (Stoppagens) getrennt vorliegen. Alternativ kann das Stoppagens entweder in der $H_2O_2$-Lösung oder in der $NO_2^-$-Lösung vorliegen. Es ist auch möglich, dass ein Teil des Stoppagens in der $H_2O_2$-Lösung und ein weiterer Teil des Stoppagens in der $NO_2^-$-Lösung vorliegt.

In einigen Ausführungsformen wird die Wirklösung auf einer zu desinfizierenden Oberfläche bis zur vollständigen Benetzung zum Zeitpunkt $t_1$ verteilt.

In einigen Ausführungsformen beträgt der Zeitraum zwischen $t_0$ und $t_1$ mindestens 5 Sekunden.

In einigen Ausführungsformen beträgt der Zeitraum zwischen $t_0$ und $t_1$ mindestens 10 Sekunden.

In einigen Ausführungsformen beträgt der Zeitraum zwischen $t_0$ und $t_1$ mindestens15 Sekunden.

Insbesondere im medizinischen Kontext enthalten Hygienevorschriften eine vorgeschriebene Verteilzeit der Wirklösung. Typischerweise werden Wirklösungen zur Handdesinfektion mindestens 30 Sekunden lang verteilt.

In einigen Ausführungsformen wirkt die Wirklösung bis zum Zeitpunkt $t_2$ zum Erhalt einer desinfizierten Oberfläche ein.

In einigen Ausführungsformen beträgt die minimale Konzentration des Stoppagens in der Wirklösung zum Zeitpunkt $t_0$ mindestens 2.5 % (v/v) und/oder die maximale Konzentration des Stoppagens in der Wirklösung < 90 % (v/v), insbesondere < 60 % (v/v), weiter insbesondere < 40 % (v/v).

In einigen Ausführungsformen beträgt die minimale Konzentration des Stoppagens in der Wirklösung zum Zeitpunkt $t_0$ mindestens 2.5 % (v/v).

Der Zeitpunkt $t_0$ ist der Zeitpunkt des Vermischens, wenn die Edukte erstmals miteinander in Kontakt kommen.

In einigen Ausführungsformen beträgt die maximale Konzentration des Stoppagens in der Wirklösung < 90 % (v/v).

In einigen Ausführungsformen beträgt die maximale Konzentration des Stoppagens in der Wirklösung < 60 % (v/v).

In einigen Ausführungsformen beträgt die maximale Konzentration des Stoppagens in der Wirklösung < 40 % (v/v).

Nach der Verteilzeit beginnt die Einwirkzeit zum Zeitpunkt $t_1$. Um mindestens 20 % mehr Edukte in der Wirklösung mit Stoppagens im Vergleich zu einer Wirklösung ohne Stoppagens zu erhalten, muss die Bedingung aus Gleichung (100) erfüllt sein.

$$\frac{c_{t_1}^{min}(x)}{c_{t_1}^{min}(0)} > 1{,}2 \quad (100),$$

wobei

$$c_{t_1}^{min}(x) = min([H_2O_2]\,(x, t{=}t_1), [NO_2^-](x, t{=}t_1)),$$

wobei x die Konzentration des Stoppagens in Volumenprozent bezogen auf das Volumen der Wirklösung zum Zeitpunkt $t{=}t_0$ ist,

$[H_2O_2]\,(x, t)$ die Konzentration an $H_2O_2$ zum Zeitpunkt t bezeichnet,

$[NO_2^-]\,(x, t)$ die Konzentration an $NO_2^-$ zum Zeitpunkt t bezeichnet.

$c_{t_1}^{min}(0)$ bezieht sich auf eine Wirklösung ohne Stoppagens. $c_{t_1}^{min}$ entspricht der maximal erzielbaren Wirksamkeit $W = \int_{t_1}^{\infty} k \cdot [H_2O_2] \cdot [NO_2^-]\, dt,$ wobei k die Geschwindigkeitskonstante der Reaktion zwischen $H_2O_2$ und $NO_2^-$ bezeichnet, d.h. das Edukt, dessen Konzentration am geringsten ist, bestimmt, wieviel biozide Wirkung maximal noch möglich ist.

In einigen Ausführungsformen liegt der pH-Wert der Wirklösung zum Zeitpunkt $t_0$ zwischen 1. und 7.

In einigen Ausführungsformen liegt der pH-Wert der Wirklösung zum Zeitpunkt $t_0$ zwischen 2 und 6.

In einigen Ausführungsformen liegt der pH-Wert der Wirklösung zum Zeitpunkt $t_0$ zwischen 3 und 5.

Für den Fall, dass der pH-Wert nicht ohne weiteres bestimmt werden kann, gilt im Kontext der vorliegenden Erfindung folgende Definition für den pH-Wert: Der pH Wert einer Lösung mit x > 0 (also mit einer Lösungmittelkonzentration (Stoppagens) > 0%) soll definiert sein als der pH-Wert, den man mit einer pH-Elektrode misst, wenn der Volumenanteil des Lösungsmittels (x) durch Wasser ersetzt wurde.

In einigen Ausführungsformen liegt die Ausgangskonzentration $[H_2O_2]_0$ zum Zeitpunkt $t_0$ zwischen 1 mM und 1000 mM.

In einigen Ausführungsformen liegt die Ausgangskonzentration $[H_2O_2]_0$ zum Zeitpunkt $t_0$ zwischen 10 mM und 500 mM.

In einigen Ausführungsformen liegt die Ausgangskonzentration $[H_2O_2]_0$ zum Zeitpunkt $t_0$, insbesondere zwischen 15 und 300 mM.

In einigen Ausführungsformen liegt die Ausgangskonzentration $[NO_2^-]_0$ zum Zeitpunkt $t_0$ zwischen 1 mM und 1000 mM.

In einigen Ausführungsformen liegt die Ausgangskonzentration $[NO_2^-]_0$ zum Zeitpunkt $t_0$ zwischen 10 mM und 500 mM.

In einigen Ausführungsformen liegt die Ausgangskonzentration $[NO_2^-]_0$ zum Zeitpunkt $t_0$ zwischen 15 und 300 mM.

Das Desinfektionsverfahren der vorliegenden Erfindung aufweisend wenigstens die Edukte $H_2O_2$ und $NO_2^-$ besteht aus mehreren Teilschritten umfassend wenigstens:

- einen Vermischungsschritt, wobei die Edukte zum Erhalt einer Wirklösung vermischt werden;
- einen Verteilungsschritt, bei dem die Wirklösung auf einer zu desinfizierenden Oberfläche verteilt wird,

wobei der Vermischungsschritt und der Verteilungsschritt in einem Verarbeitungszeitraum $Z_A$ stattfinden, der zum Zeitpunkt $t_0$ beginnt, wenn die Edukte zuerst miteinander in Kontakt gebracht werden, und zum Zeitpunkt $t_1$, bei dem jeder Punkt auf der zu desinfizierenden Oberfläche mit Wirklösung benetzt ist, endet, wobei $t_0$ gleich 0 ist und $t_1$ größer als $t_0$ ist und

- darauffolgend einen Einwirkschritt, bei dem die verteilte Wirklösung über einen Einwirkzeitraum $Z_E$, der zum Zeitpunkt $t_1$ beginnt und nach der Zeitspanne $Z_E$ zum Zeitpunkt $t_2$ endet, auf der mit Wirklösung kontaktierten Oberfläche einwirkt,

wobei $t_2$ den Zeitpunkt darstellt, an dem jeder Punkt auf der mit Wirklösung kontaktierten Oberfläche ausreichend lange mit Wirklösung benetzt ist, um eine desinfizierende Wirkung zu erhalten, und wobei $t_2$ größer als $t_1$ ist, die zeitintegrierte Reaktionsrate W über den Einwirkzeitraum $Z_E$ durch das Integral

$$W = \int_{t_1}^{t_2} k_1 \cdot [H_2O_2] \cdot [NO_2^-]\, dt \geq 10\ mM \qquad (5)$$

dargestellt wird,

wobei $t_1$ und $t_2$ wie oben definiert sind, und
wobei $[H_2O_2]$ und $[NO_2^-]$ die Konzentrationen der Edukte während des Einwirkzeitraums $Z_E$ bezeichnen, und
wobei $k_1$ die pH-Wert abhängige Geschwindigkeitskonstante der Reaktion zwischen $H_2O_2$ und $NO_2^-$ bzw. $HNO_2$ bezeichnet, und
wobei der pH-Wert und die Temperatur eine Zeitabhängigkeit aufweisen können, und In einigen Ausführungsformen ist die die maximale $NO_2^-$ Konzentration zum Zeitpunkt $t_0$ *des Vermischungsschritts* bei 300 mM.

In einigen Ausführungsformen übersteigt $t_2$ 3 Minuten nicht,

In einigen Ausführungsformen liegt der pH-Wert der Wirklösung vor Kontakt mit der zu desinfizierenden Oberfläche im Bereich von $2{,}1 \leq pH < 6{,}8$.

Die pH-Wert-abhängige Geschwindigkeitskonstante $k_1$ kann folgendermaßen berechnet werden:

$$k_1 = k_4 \frac{[H_3O^+]^2}{\left(K_{S,H_3O_2^+} + [H_3O^+]\right)\left(K_{S,HNO_2} + [H_3O^+]\right)} \tag{6}$$

mit

$$k_4 = 3{,}56 \cdot 10^{14} \exp\left(-\frac{E_A}{RT}\right) M^{-1}s^{-1} \tag{7}$$

$$K_{S,HNO_2} = 5{,}13 \times 10^{-4} \tag{8}$$

$$K_{S,H_3O_2^+} = 2 \times 10^{-2} \tag{9}$$

und der einheitenlosen Größe

$$[H_3O^+] = 10^{-pH} \tag{10}$$

mit der effektiven Aktivierungsenergie $E_A$=70 kJ/mol und der Temperatur T. Bei 20 °C beträgt $k_4$ 120 $M^{-1}s^{-1}$.

Die zeitabhängigen Konzentrationen der Edukte $NO_2^-$ und $H_2O_2$ können während des Einwirkzeitraums mittels folgender Gleichungen berechnet werden:

$$[NO_2^-] = \frac{A}{k_1}, \tag{11}$$

$$[H_2O_2] = \frac{A+D}{k_1+rk_1}, \tag{12}$$

mit

$$A = -\frac{D}{1-\exp(D(t-C))} \tag{13}$$

$$C = -\frac{\ln\left(\frac{D}{[NO_2^-]_0 \cdot k_1}+1\right)}{D} \tag{14}$$

und

$$D = [H_2O_2]_0 \cdot (k_1 + rk_1) - [NO_2^-]_0 \cdot k_1, \tag{15}$$

mit $k_1$, $k_4$, $K_{S,HNO_2}$, $K_{S,H_3O_2^+}$ und $[H_3O^+]$ wie oben beschrieben.

$[H_2O_2]_0$ und $[NO_2^-]_0$ bezeichnen die initialen Anfangskonzentrationen zum Zeitpunkt des Vermischungsschrittes von $H_2O_2$ und $NO_2^-$ in der Wirklösung. Diese werden durch die Eduktkonzentrationen und die Art der Vermischung bzw. Verdünnung vorgegeben. Beispielsweise erhält man im Falle einer Eduktkonzentration von 200 mM $H_2O_2$ in Eduktlösung 1 und 200 mM $NO_2^-$ in Eduktlösung 2 und einem Vermischungsverhältnis von 1:1 initiale Konzentrationen von

$$[H_2O_2]_0 = [NO_2^-]_0 = 100 \text{ mM}.$$

Zudem ist

$$r = 0{,}11, \tag{16}$$

wobei $r$ ein Ausgasungskoeffizient ist, der die Bildung von $NO_x$ aus $NO_2^-$ beschreibt und weiter unten näher beschrieben wird.

Da die Ausgangsstoffe ($NO_2^-$ und $H_2O_2$) mit der Zeit umgesetzt werden, nimmt die effektive Reaktionsrate der Reaktion zwischen $H_2O_2$ und $NO_2^-$ stetig ab. Aufgrund der kurzen Halbwertszeit der Reaktionsprodukte werden diese nicht akkumuliert und somit ist die momentane Reaktionsrate von $H_2O_2$ und $NO_2^-$ für die Wirksamkeit der Wirklösung zu einem gegebenen Zeitpunkt während der Einwirkzeit maßgeblich. Für den Einsatz der Wirklösung als Desinfektionsmittel ist es notwendig, dass die Wirksamkeit für eine definierte minimale Wirkdauer gegeben ist. Daher darf die zeitintegrierte Reaktionsrate W einen minimalen Wert nicht unterschreiten. Die heuristische Formel (5) erlaubt es, für Dekontaminationsanwendungen bei einer bestimmten Prozesstemperatur anwendbare Konzentrationen von $H_2O_2$ und $NO_2^-$ und einen entsprechenden pH-Wert zu wählen.

Im Gegensatz zu vegetativen Bakterien sind Bakteriensporen und unbehüllte Viren mit alkoholbasierten Mitteln nicht oder nur nach unzureichend langer Zeit inaktivierbar. Bei einer Reaktionsrate W $\geq$ 10 mM werden nicht nur vegetative Bakterien, sondern auch Bakteriensporen inaktiviert.

In einer Ausführungsform ist die zeitintegrierte Reaktionsrate W der Reaktion zwischen $H_2O_2$ und $NO_2^-$ größer oder gleich 17.

Bei einer Reaktionsrate W $\geq$ 17 mM werden neben vegetativen Bakterien und Bakteriensporen auch unbehüllte Viren inaktiviert.

In einer Ausführungsform, die sich ausschließlich auf vegetativen Bakterien richtet ist W=0,3, insbesondere 0,5.

Eine höhere zeitintegrierte Reaktionsrate W erhöht die desinfizierende Wirkung auf der mit Wirklösung kontaktierten Oberfläche.

Der Verarbeitungszeitraum $Z_A$ umfasst den Vermischungsschritt und den Verteilungsschritt, wobei der Verteilungsschritt zeitgleich mit dem Vermischungsschritt zum Zeitpunkt $t_0=0$ beginnen kann, oder dem nachgestellt sein kann. Der Verarbeitungszeitraum beginnt bei $t_0=0$.

Weiterhin können auch vor dem Verarbeitungszeitraum, also vor dem Zeitpunkt $t_0=0$ relevante Schritte erfolgen, wie z.B. ein Verdünnungsschritt. Diese Schritte sind aber für das Zeitintervall zur Berechnung der zeitintegrierten Reaktionsrate nicht relevant und können daher vor $t_0=0$ liegen.

Der Verarbeitungszeitraum muss ausreichend groß sein, um jeden Punkt der zu desinfizierenden Oberfläche mit Wirklösung zu benetzen. Gleichzeitig sollte der Verarbeitungszeitraum aber auch nicht zu groß gewählt sein, dass nach Verteilung der Wirklösung auf der zu desinfizierenden Oberfläche noch ausreichend reaktive Wirklösung vorhanden ist, um eine desinfizierende Wirkung zu erzielen und der notwendige Anteil an Stoppagens gleichzeitig relativ niedrig ist.

In einigen Ausführungsformen ist der Verarbeitungszeitraum, der zum Zeitpunkt $t_1$ endet, ausgewählt ist aus einem Bereich von $0 < t_1 \leq 75$ s, insbesondere ausgewählt ist aus dem Bereich $0 < t_1 \leq 30$ s, insbesondere ausgewählt ist aus einem Bereich $0 < t_1 \leq 15$s, insbesondere ausgewählt ist aus einem Bereich $0 < t_1 \leq 2$s.

In einigen Ausführungsformen beginnt die Einwirkzeit nach 2s.

In einigen Ausführungsformen beginnt die Einwirkzeit nach 15s.

In einigen Ausführungsformen beginnt die Einwirkzeit nach 30s.

In einigen Ausführungsformen beginnt die Einwirkzeit nach 75s.

In einigen Ausführungsformen ist ein längerer Verarbeitungszeitraum notwendig, der zum Zeitpunkt $t_1$ endet, wobei dieser ausgewählt ist aus einem Bereich von $15 < t_1 \leq 75$ s, insbesondere ausgewählt ist aus dem Bereich $30 < t_1 \leq 75$ s, insbesondere ausgewählt ist aus einem Bereich $50 < t_1 \leq 75$s.

In einigen Ausführungsformen ist ein kürzerer Verarbeitungszeitraum notwendig, der zum Zeitpunkt $t_1$ endet, wobei dieser ausgewählt ist aus einem Bereich von $0 < t_1 \leq 30$ s, insbesondere ausgewählt ist aus dem Bereich $0 < t_1 \leq 15$ s, insbesondere ausgewählt ist aus einem Bereich $0 < t_1 \leq 2$s.

In einigen Ausführungsformen ist ein Verarbeitungszeitraum notwendig, der zum Zeitpunkt $t_1$ endet, wobei dieser ausgewählt ist aus einem Bereich von $2 < t_1 \leq 75$ s, insbesondere ausgewählt ist aus dem Bereich $2 < t_1 \leq 30$ s, insbesondere ausgewählt ist aus einem Bereich $2 < t_1 \leq 15$s.

Weiterhin sollte der Zeitbereich (Summe aus $Z_A$ und $Z_E$), insbesondere bei Anwendungen in einer Handdesinfektion ausreichend kurz gewählt sein, um die notwendige desinfizierende Wirkung in einem noch angemessenen Bereich zu erzielen. Eine zu lange Zeitspanne, wie beispielsweise über 10 Minuten, ist bei einer Händedesinfektion, selbst im klinischen Bereich, weder praktikabel noch sinnvoll anwendbar.

Die Vermischung der Edukte $H_2O_2$ und $NO_2^-$ zur Herstellung der Wirklösung kann vor Kontakt mit der zu desinfizierenden Oberfläche stattfinden, oder direkt auf der zu desinfizierenden Oberfläche erfolgen. Der Vermischungsschritt kann ohne äußere Einwirkung durch Diffusion und Konvektion erfolgen, durch mechanische Verteilung unterstützt werden, oder in einem Sprühprozess, bei dem die Edukte gemeinsam auf die zu desinfizierende Oberfläche gesprüht werden, integriert sein.

Des Weiteren spielt der *pH*-Wert eine entscheidende Rolle in dem erfindungsgemäßen Desinfektionsverfahren.

In einigen Ausführungsformen liegt der *pH*-Wert der Wirklösung auf der mit Wirklösung kontaktierten Oberfläche im Bereich von $2{,}1 \leq pH \leq 6{,}8$, insbesondere in einem Bereich von $2{,}5 \leq pH \leq 5$, und insbesondere in einem Bereich von $3{,}3 \leq pH \leq 4{,}7$.

Die Reaktionsrate der Reaktion zwischen $H_2O_2$ und $NO_2^-$ hängt gemäß (6) vom *pH*-Wert der Lösung ab. Mit fallendem *pH*-Wert, also mit steigender Konzentration an $H_3O^+$, nimmt die Reaktionsrate $k_1$ zu. Bei niedrigen *pH*-Werten ist daher die desinfizierende Wirkung der Wirklösung höher, allerdings erlauben niedrige *pH*-Werte auf Grund der hohen Reaktionsrate von $H_2O_2$ und $NO_2^-$ in Kombination mit der Kurzlebigkeit der entstehenden Reaktionsprodukte keinen ausreichend großen Verarbeitungs- und Einwirkzeitraum. Bei höheren *pH*-Werten verringert sich die Reaktionsrate von $H_2O_2$ und $NO_2^-$ signifikant, wodurch allerdings auch die desinfizierende Wirkung der Wirklösung abnimmt.

Im Gegensatz zur Dekontamination in Suspensionen wurde festgestellt, dass eine Ansäuerung bei der Dekontamination von Oberflächen zu einer signifikanten Verschlechterung der Wirkung führen kann. Dies resultiert aus der Notwendigkeit, dass die Flüssigkeit auf der Oberfläche in einem Verteilungsschritt auf der Oberfläche aufgebracht und/oder verteilt werden muss und bei strukturierten und porösen Oberflächen durch Diffusion in die Oberfläche eindringen muss. Die Wirklösung darf ihre Desinfektionswirkung während dieser Zeit nicht verlieren, was durch einen zu niedrigen *pH*-Wert jedoch bewirkt wird. In diesem Fall werden die Edukte zu schnell abgebaut, bevor sie an jeder Stelle auf der Oberfläche ihre antimikrobielle Wirkung entfalten können. Dieses Problem wird durch die vorliegende Erfindung für eine Wirklösung aus mindestens $NO_2^-$ und $H_2O_2$ gelöst, indem ein *pH*-Wert-Bereich identifiziert wird, in welchem der Einsatz als Mittel zur Oberflächendesinfektion möglich ist.

Viele Oberflächen haben selbst eine *pH*-Wert regulierende Eigenschaft, insbesondere eine Pufferwirkung, wie zum Beispiel die Hautoberfläche. Der *pH*-Wert, der für das Verfahren der vorliegenden Erfindung ausschlaggebend ist, ist deshalb der *pH*-Wert, der sich auf der mit Wirklösung benetzten Oberfläche ergibt. Dem Fachmann sind solche puffernden Oberflächen und ihre puffernde Wirkung bekannt.

In einer Ausführungsform der vorliegenden Erfindung soll das Desinfektionsverfahren zur Desinfektion einer den *pH*-Wert stark puffernden Oberfläche, insbesondere Haut, Wunden oder anderen organischen Oberflächen, angewendet werden, wobei sich ein geeigneter *pH*-Wert auf der Oberfläche dadurch ergibt, dass der pH-Wert der Wirklösung vor Kontakt mit der zu desinfizierenden Oberfläche im Bereich von 2,1 bis 4,5, insbesondere in einem Bereich von 2,1 bis 3,6, insbesondere in einem Bereich von 2,1 bis 3,2 liegt.

Dieser *pH*-Wert wird durch den Kontakt mit der puffernden Oberfläche in Abhängigkeit der Oberflächenbeschaffenheit um 0,2 bis 1,7, insbesondere um 0,2 bis 0,8 angehoben.

Dadurch ist der *pH*-Wert der Wirklösung geringer als der *pH*-Wert der Wirklösung auf der zu desinfizierenden Oberfläche. Durch diese Eigenschaft reagieren die Edukte außerhalb der zu desinfizierenden Oberfläche schnell ab und akkumulieren nicht in der Umwelt. Auf der zu desinfizierenden Oberfläche, insbesondere bei der Oberfläche eines Körperteils, insbesondere einer Hand, hingegen läuft die Reaktion zwischen $H_2O_2$ und $NO_2^-$ durch die Pufferwirkung der Oberfläche etwas langsamer ab, wodurch sich die desinfizierende Wirkung entfalten kann. Somit bleibt die desinfizierende Wirkung auf der bestimmungsgemäßen Oberfläche ausreichend lang wirksam, auf nicht bestimmungemäßen Oberflächen erfolgt ein schneller Abbau der Edukte und somit keine Akkumulation.

In einer Ausführungsform sind die Ausgangsstoffmengen der Edukte identisch, insbesondere bei Anwendungen wenn Ausgasen von $NO_x$ vernachlässigbar ist, somit werden $NO_2^-$ und $H_2O_2$ vollständig umgesetzt. Dadurch werden keine bioziden Wirkstoffe in die Umwelt abgegeben.

In einer Ausführungsform beträgt die Effizienz $E = W/W_{max}$ des Verfahrens bei Prozessbedingt vorgegebenen Zeiten $t_1$ und $t_2$ mindestens 10 %, insbesondere mindestens 20 %, insbesondere mindestens 30 %, wobei

$$W_{max} = \min(\,[H_2O_2]_0, [NO_2^-]_0\,)\,(\exp(-Gt_1) - \exp(-Gt_2)) \qquad (17)$$

mit $G = \ln\left(\frac{t_2}{t_1}\right)/(t_2 - t_1)$ den $t_1$ maximal erreichbaren Wirksamkeitsparameter bezeichnet und

$\min(\,[H_2O_2]_0, [NO_2^-]_0\,)$ die minimale Konzentration ausgewählt aus den initialen Konzentrationen $[H_2O_2]_0$ und

$[NO_2^-]_0$ bezeichnet. Dies stellt sicher, dass die verwendeten Edukte durch sinnvolle Wahl der wählbaren Prozessparameter pH-Wert, Temperatur und Ausgangskonzentrationen der Edukte effizient eingesetzt werden.

In einer Ausführungsform unterscheiden sich die Ausgangsstoffmengen an Edukten um weniger als 10 % voneinander, insbesondere die Ausgangsstoffmenge von $NO_2^-$ ist um 2 % bis 10 % höher als die Ausgangsstoffmenge von $H_2O_2$. Der genaue Wert ist für eine gegebene Anwendung, also für eine gegebene Oberfläche und Flüssigkeitsmenge, zu bestimmen. In diesem Fall werden als stabile Endprodukte ausschließlich Nitrat und Wasser gebildet, während $NO_2^-$ und $H_2O_2$ vollständig umgesetzt werden. Dadurch werden keine bioziden Wirkstoffe in die Umwelt abgegeben. Das erfindungsgemäße Desinfektionsverfahren ist somit besonders umweltfreundlich.

Eine geringfügig höhere Ausgangsstoffmenge an $NO_2^-$ im Vergleich zu $H_2O_2$ ist hilfreich, um den Verlust an wirksamem $NO_2^-$ durch Ausgasen von $NO_x$ vorzubeugen.

Das Ausgasen von $NO_x$ ist bei einer Oberflächendesinfektion auf Grund der größeren Oberfläche deutlich stärker als bei einer Desinfektion, die in Lösung oder Suspension stattfindet. Beim Verteilen der Wirklösung auf einer Oberfläche entsteht nur ein dünner Flüssigkeitsfilm, bei dem große Anteile des eingesetzten $NO_2^-$ als gasförmige Stickoxide ($NO_x$) freigesetzt werden können. Dies hat unter anderem zur Folge, dass bis zu 10% des in die Flüssigkeit eingebrachten $NO_2^-$ in Form von $NO(g)$ oder insbesondere $NO_2(g)$ ausgasen. Die Ausgasung führt zu einem beschleunigten Abbau von $NO_2^-$ in einer Wirklösung aus $H_2O_2$ und $NO_2^-$ auf Oberflächen, verglichen mit einer identisch angesetzten Wirklösung in Suspension. Die Ausgasung beeinflusst die Reaktionskinetik und sollte auch aus gesundheitlichen Gründen niedrig gehalten werden.

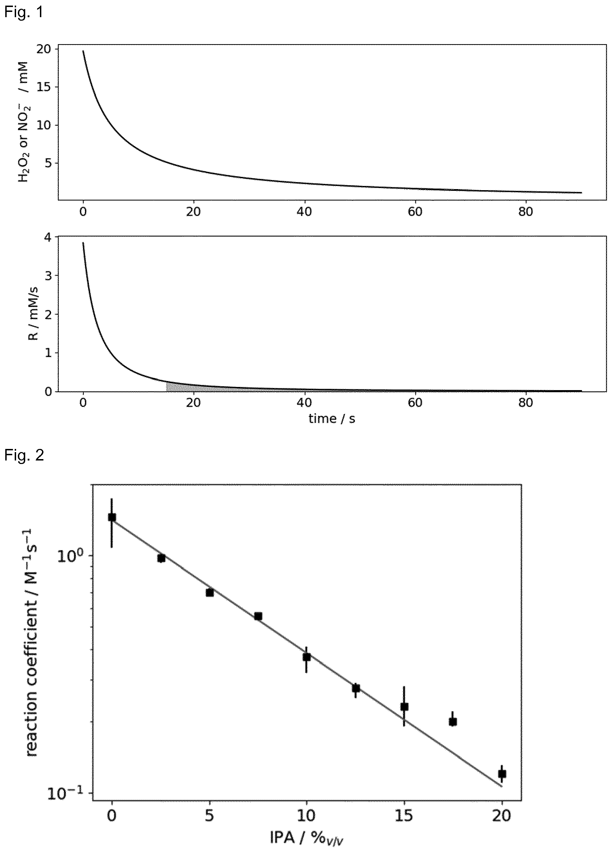

Die $NO_x$-Emissionen sind auf zwei grundlegende Prozesse zurückzuführen: Einerseits kann der Einsatz einer Säure zur Einstellung des *pH*-Wertes direkt eine $NO_x$-Ausgasung bewirken, so beispielsweise der in Fig. 1 dargestellte Prozess:

$$HNO_2 + HNO_2 \rightarrow NO + NO_2 + H_2O \rightarrow NO(g) + NO_2(g) + H_2O. \qquad (18)$$

Für den Prozess (18) ist die Anwesenheit von $H_2O_2$ nicht erforderlich. Andererseits kann die Bildung von ONOOH, welche die Anwesenheit von $H_2O_2$ erfordert, durch die Reaktion (19)

$$ONOOH \rightarrow NO_2 + OH \qquad (19)$$

durch anschließende Ausgasung von $NO_2$ zu den $NO_x$-Emissionen beitragen.

Die Ausgasung von $NO_x$ wurde mittels Experimenten und Computersimulationen untersucht. In einer Ausführung der vorliegenden Erfindung wird die Ausgasung als Ausgasungsrate durch die Formel

$$R_{degas} = R_1 \times r \qquad (20)$$

beschrieben, welche proportional zur effektiven Vernichtungsrate $R_1$ von $NO_2^-$ und $H_2O_2$ durch Reaktion (1) angenommen werden kann, und zur Vernichtung von $NO_2^-$ entsprechend der Formel

$$\frac{d[NO_2^-]}{dt} = -R_1 - R_{degas} = -k_1[H_2O_2][NO_2^-] \times (1+r) \qquad (21)$$

beiträgt. Hierbei bezeichnet r einen auf $R_1$ bezogenen Anteil, welcher zur Ausgasung von $NO_2^-$ in Form von $NO_x$ führt. Diese Form resultiert daraus, dass die Reaktion

$$ONOOH \rightarrow NO_2 + OH \qquad (22)$$

den wesentlichen Beitrag zur Ausgasung bei der Oberflächendekontamination liefert. Während *r* in Suspensionsversuchen üblicherweise vernachlässigbar klein, etwa im Bereich von $r \leq 0.01$ ist, kann *r* bei der Oberflächendekontamination Werte im Bereich $r \leq 0.11$ annehmen. Der konkrete Wert hängt von der jeweiligen Anwendung, insbesondere von der Schichtdicke des Flüssigkeitsfilms, ab. Die auftretende Ausgasung beeinflusst also bei einer Oberflächendesinfektion die Reaktionskinetik der Reaktion von $H_2O_2$ und $NO_2^-$, was bei einer Desinfektion in Suspension zu vernachlässigen wäre.

Eine Möglichkeit, die Menge an ausgasenden $NO_x$, zu begrenzen, ist die Ausgangskonzentration von $NO_2^-$ zu

begrenzen.

In einigen Ausführungsformen wird somit eine maximale Ausgangskonzentration an $NO_2^-$ zum Zeitpunkt $t_0$ eine Konzentration von 300 mM, insbesondere von 200 mM, insbesondere von 100 mM nicht überschritten.

Das erfindungsgemäße Desinfektionsverfahren kann zur Desinfektion von Oberflächen eingesetzt werden. Das erfindungsgemäße Desinfektionsverfahren kann insbesondere zur Desinfektion von Haut und/oder zur Desinfektion von Wunden verwendet werden.

Das Desinfektionsverfahren der vorliegenden Erfindung kann weiterhin eingesetzt werden zur Dekontamination von Medizinprodukten, insbesondere von thermolabilen Medizinprodukten wie Schläuche von Endoskopen sowie von Behältern und Wannen.

Das Desinfektionsverfahren der vorliegenden Erfindung kann weiterhin eingesetzt werden zur Dekontamination von Saatgut, Nutzpflanzen, tierischen Produkten, Lebensmitteln, Verpackungen sowie von Getränkebehältern oder Getränkeleitungen.

In einer Ausführungsform des erfindungsgemäßen Desinfektionsverfahrens können den Edukten und/oder der Wirklösung Säurepuffer bzw. Säurepufferlösungen zugesetzt werden. Als Puffer können beispielsweise Citratpuffer, Essigsäure-Acetat-Puffer, Phosphat-CitratPuffer, Phosphat-Puffer oder Citratpuffer eingesetzt werden. Citrathaltige Pufferlösungen eignen sich insbesondere aufgrund ihres angenehmen Geruchs.

In einer Ausführungsform des erfindungsgemäßen Desinfektionsverfahrens können den Edukten vor und/oder während des Vermischungsschrittes Zusatzstoffe hinzugefügt werden. Denkbare Zusatzstoffe umfassen unter anderem Lösungsmittel, Basen, Duftstoffe, Farbstoffe und/oder weitere Desinfektionsmittel, und/oder Ozon.

Des Weiteren können Suspensionen mit nicht-wasserlöslichen Stoffen erzeugt werden, insbesondere durch Beimischungen von Fetten und Tensiden.

In einer weiteren Ausführungsform können eine oder mehrere Plasmaquellen genutzt werden, um eines oder mehrere der Edukte herzustellen.

Somit wäre es möglich, die Edukte $H_2O_2$ und $NO_2^-$ aus Luft und Wasser mittels Strom herzustellen. Im Stand der Technik sind Plasmaverfahren ausreichend offenbart, so dass der Fachmann ein entsprechendes Plasma auswählen kann .

In einer weiteren Ausführung der vorliegenden Erfindung wird durch das Plasma zusätzlich Ozon hergestellt, welches Teil der Wirklösung sein kann.

Das Verfahren zur Desinfektion von Oberflächen, das Gegenstand dieser Erfindung ist, zeichnet sich dadurch aus, dass es eine sporizide Wirkung hat. Es gibt in Deutschland kein zugelassenes Desinfektionsmittel zur Desinfektion von Haut, dass auch gegenüber Bakteriensporen eine desinfizierende Wirkung hat. Weiterhin ist das erfindungsgemäße Verfahren, geruchsarm und ist vorteilhaft gegenüber üblichen Desinfektionsverfahren zur Desinfektion von Haut, weil es die Haut nicht austrocknet.

Ein weiterer Aspekt der Erfindung stellt eine Vorrichtung zur simultanen Abgabe von zumindest zwei Volumenströmen von $H_2O_2$ und $NO_2^-$ Lösungen, insbesondere von zumindest zwei Volumenströmen gleicher Größe, bereit, umfassend zumindest zwei Reservoirs zur Aufnahme von $H_2O_2$ und zur Aufnahme von $NO_2^-$, und in einem jeweiligen Reservoir angeordnet einen verschiebbaren Kolben zur Förderung eines Fluids aus dem jeweiligen Reservoir, wobei die Kolben über eine Kraftübertragungseinrichtung derart miteinander gekoppelt sind, dass sie synchron parallel zueinander verschiebbar sind, so dass die Fluide aus den Reservoirs zur selben Zeit, insbesondere mit gleichen Volumenströmen, ausbringbar sind.

Das Stoppagens kann entweder in einem oder beiden Volumenströmen von $H_2O_2$ und $NO_2^-$ enthalten sein oder über einen dritten Volumenstrom hinzugefügt werden.

Die Kolben sind gleichzeitig mit der gleichen Geschwindigkeit verschiebbar. D.h., dass die Kolben auch das gleiche Beschleunigungsverhalten haben, sodass mit ihnen vollkommen gleichartige Bewegungsabläufe auf zwei parallel zueinander angeordneten Bewegungsbahnen realisierbar sind, sodass die Fluide mit einem festen Mischungsverhältnis gleichzeitig ausgebracht werden können.

Insbesondere können die beiden Reservoirs in einer gemeinsamen Kartusche angeordnet sein.

In einer Ausführungsform ist die Vorrichtung zur simultanen Abgabe von zumindest zwei Volumenströmen von $H_2O_2$ und $NO_2^-$ dadurch gekennzeichnet, dass die beiden Reservoirs durch zumindest eine gemeinsame Trennwand voneinander abgeteilt sind, wobei diese Trennwand eine geringere Biegefestigkeit aufweist als die an der Trennwand gleitenden Seiten der Kolben, und wobei ein erster Kolben einen in Richtung auf einen zweiten Kolben vorstehenden Vorsprung aufweist und der zweite Kolben eine im Wesentlichen hinsichtlich Form und Größe des Vorsprungs komplementäre Aussparung aufweist, sodass bei Verschiebung eines Kolben der jeweils andere Kolben unter Verformung der Trennwand über einen mittelbaren mechanischen Eingriff des Vorsprungs in der Aussparung mitgenommen wird.

Der mechanische Eingriff erfolgt dabei lediglich mittelbar, da zwischen dem Vorsprung und der Aussparung weiterhin ein Bereich der abschnittsweise, insbesondere als Membran ausgestalteten, verformten Trennwand angeordnet ist.

In einer Ausführungsform ist die Vorrichtung zur simultanen Abgabe von zumindest zwei Volumenströmen von

$H_2O_2$ und $NO_2^-$ dadurch gekennzeichnet, dass die Reservoirs durch zumindest eine gemeinsame Trennwand voneinander abgeteilt sind, wobei die Kolben über wenigstens ein Verbindungselement verbunden sind, welches dazu eingerichtet ist, bei einer Verschiebung der Kolben die dazwischen befindliche Trennwand zumindest abschnittsweise zu zerschneiden.

Zu diesem Zweck ist das Verbindungselement vorzugsweise mit einem Keilsegment bzw. einer Schneide oder Klinge ausgestattet, mit der es auch bei geringer Krafteinwirkung auf die Kolben möglich ist, die insbesondere als Membran ausgestaltete Trennwand einzuschneiden bzw. zu zerschneiden.

In einer Ausführungsform befindet sich das Verbindungselement mit der Schneide an der Seite der Kolben, die den jeweiligen Auslässen aus den Reservoirs bezogen auf die Bewegungsachse der mittels des Verbindungselements realisierten Kolben-Einheit gegenüberliegt.

In einer Ausführungsform ist die Vorrichtung zur simultanen Abgabe von zumindest zwei Volumenströmen von $H_2O_2$ und $NO_2^-$ dadurch gekennzeichnet, dass jedes Reservoir einen Auslass aufweist, wobei an einem jeweiligen Auslass eine Fluidleitung angeschlossen ist, welche mit einer Mischeinheit zur Mischung der Fluide aus den Reservoirs strömungstechnisch verbunden ist.

Eine derartige Mischeinheit kann zum Beispiel ein sogenanntes T-Stück sein, in welchem die Fluide aus zwei Reservoirs zusammengeführt werden.

In einer weiteren vorteilhaften Ausgestaltung der erfindungsgemäßen Vorrichtung ist vorgesehen, dass im Strömungspfad zwischen einem jeweiligen Auslass und der Mischeinheit jeweils ein Rückschlagventil angeordnet ist zur Verhinderung einer Rückströmung gemischten Fluides in die Reservoirs.

In einer Ausführungsform ist die Vorrichtung zur simultanen Abgabe von zumindest zwei Volumenströmen von $H_2O_2$ und $NO_2^-$ dadurch gekennzeichnet, dass an die Mischeinheit strömungstechnisch eine erste Pumpe zur Erzeugung eines Unterdrucks und somit zur Förderung der Mischung der Fluide aus der Mischeinheit angeschlossen ist.

In einer Ausführungsform ist die Vorrichtung zur simultanen Abgabe von zumindest zwei Volumenströmen von $H_2O_2$ und $NO_2^-$ dadurch gekennzeichnet, dass die Vorrichtung eine Auslasseinrichtung, insbesondere eine Düse aufweist, mit der die Fluide als Gemisch in flüssiger Form oder auch als Sprühnebel ausgebbar sind.

In einer Ausführungsform ist die Vorrichtung zur simultanen Abgabe von zumindest zwei Volumenströmen von $H_2O_2$ und $NO_2^-$ dadurch gekennzeichnet, dass die Kraftübertragungseinrichtung je Kolben ein Kraftübertragungselement aufweist, mit denen jeweils ein Kolben mit einer Kraft zwecks Verschiebung des Kolbens beaufschlagbar ist, wobei die beiden Kraftübertragungselemente mechanisch miteinander gekoppelt sind.

Insbesondere kann diese mechanische Kopplung an bzw. durch eine Vorschubeinrichtung realisiert sein, mit welcher jeweils eine Kraft auf ein jeweiliges Kraftübertragungselement ausübbar ist, welches diese Kraft wiederum auf den jeweiligen Kolben überträgt.

In einer Ausführungsform ist die Vorrichtung zur simultanen Abgabe von zumindest zwei Volumenströmen von $H_2O_2$ und $NO_2^-$ dadurch gekennzeichnet, dass die Kraftübertragungseinrichtung wenigstens einem Kolben zugeordnet und mit diesem strömungstechnisch gekoppelt, insbesondere von diesem zumindest bereichsweise einseitig begrenzt, eine Druckkammer aufweist, mit der eine zweite Pumpe zur Erzeugung eines Überdrucks strömungstechnisch in Verbindung steht, sodass bei Betätigung der zweiten Pumpe und Erzeugung eines Überdrucks der jeweilige Kolben verschoben wird.

Das bedeutet, dass der Kolben an seiner dem auszugebenden Fluid gegenüberliegenden Seite fluidechnisch mit der zweite Pumpe zur Erzeugung eines Überdrucks strömungstechnisch in Verbindung steht, sodass bei Erzeugung des Überdrucks an der dem Fluid abgewandten Seite des Kolbens dieser Kolben verschoben wird und entsprechend das Fluid aus dem ihm zugeordneten Reservoir heraus fördert.

Dabei kann mehreren Kolben eine gemeinsame Druckkammer zugeordnet sein.

In einer Ausführungsform ist die Vorrichtung zur simultanen Abgabe von zumindest zwei Volumenströmen von $H_2O_2$ und $NO_2^-$ dadurch gekennzeichnet, dass die Vorrichtung drei Reservoirs aufweist, wobei in dem dritten Reservoir ein verschiebbarer dritter Kolben zur Förderung eines Fluids aus dem dritten Reservoir angeordnet ist, wobei die drei Kolben über eine Kraftübertragungseinrichtung derart miteinander gekoppelt sind, dass sie synchron parallel zueinander verschiebbar sind, so dass die Fluide aus den Reservoirs mit gleichen Volumenströmen ausbringbar sind.

In einer Ausführungsform ist die Vorrichtung zur simultanen Abgabe von zumindest zwei Volumenströmen von $H_2O_2$ und $NO_2^-$ dadurch gekennzeichnet, dass zumindest ein erstes Reservoir an wenigstens zwei Seiten von wenigstens einem weiteren Reservoir benachbart ist, wobei in dem weiteren Reservoir das Fluid eine geringere Transluzenz aufweist als das Fluid im ersten Reservoir zwecks Verringerung der Lichteinstrahlung in das Fluid im ersten Reservoir.

In einer besonderen Ausführungsform ist vorgesehen, dass das erste Reservoir vollständig von zwei weiteren Reservoirs umgeben ist.

In einem weiteren Aspekt der Erfindung wird ein Verfahren zur simultanen Abgabe von zumindest zwei Volumenströmen von $H_2O_2$ und $NO_2^-$, insbesondere von zumindest zwei Volumenströmen gleicher Größe, bereitgestellt, bei dem zumindest zwei Reservoirs umfassend $H_2O_2$ und $NO_2^-$ zur Verfügung gestellt werden, und in einem jeweiligen Reservoir ein Kolben zur Förderung eines jeweiligen Fluids aus dem jeweiligen Reservoir verschoben wird, wobei die Kolben über eine Kraftübertragungseinrichtung derart miteinander gekoppelt sind, dass sie synchron parallel zueinander verschoben werden, so dass die Fluide aus den Reservoirs zur selben Zeit, insbesondere mit gleichen Volumenströmen, ausgebracht werden.

In Ausführungsformen mit nur zwei Reservoirs befindet sich das Stoppagens in einem oder beiden Volumenströmen von $H_2O_2$ und $NO_2^-$. Bei Vorhandensein eines dritten Reservoirs kann das Stoppagens alternativ oder zusätzlich über dieses Reservoir zugegeben werden.

**Bezugszeichenliste:**

| | |
|---|---|
| 1 | Vorrichtung |
| 2 | Kartusche |
| 3 | Öffnung |

| | |
|---|---|
| 10 | Volumenstrom zum ersten Reservoir |
| 11 | Volumenstrom zum zweiten Reservoir |
| 20 | erstes Reservoir |
| 21 | zweites Reservoir |
| 22 | drittes Reservoir |
| 23 | äußeres Reservoir |
| 24 | inneres Reservoir |
| 25 | Trennwand |
| 26 | Biegezone |

| | |
|---|---|
| 30 | erster Kolben |
| 31 | zweiter Kolben |
| 32 | dritter Kolben |
| 33 | Vorsprung |
| 34 | Aussparung |
| 35 | Vorschub der Kolben |
| 36 | Kraftübertragungselemente |

| | |
|---|---|
| 50 | Verbindungselement |
| 51 | Klinge |

| | |
|---|---|
| 60 | erste Pumpe |
| 61 | Rückschlagventile |
| 62 | Mischeinheit |
| 63 | Düse |
| 64 | Druckkammer |
| 65 | zweite Pumpe |

Kurze Beschreibung der Figuren

Figur 1 zeigt den Konzentrationsverlauf (oben) und die Reaktionsrate entsprechend Gleichung (3) (unten) mit $[H_2O_2]_0$ = $[NO_2^-]_0$ = 20 mM sowie einem pH-Wert von 3,3 und einer Temperatur von 37 °C. Der gefüllte Bereich illustriert das Integral über die Einwirkzeit von $t_1$ = 15 s bis $t_2$ = 75 s.

Figur 2 zeigt den Einfluss der Isopropanolkonzentration IPA auf den Reaktionskoeffizienten der Reaktion (2) bei 20 °C.

Figur 3 zeigt die Isopropanolkonzentration einer zu Beginn 50 %igen Isopropanollösung nach 0 s, 30 s und 60 s auf einer auf 37 °C geheizten Metallplatte.

Figur 4 zeigt den angenommenen Konzentrationsverlauf von Isopropanol (oben), den berechneten Konzentrations-

verlauf von $H_2O_2$ und $NO_2^-$ (mitte) und die Reaktionsrate (unten) entsprechend Gleichung (3) mit Geschwindigkeits-konstante (9) und Anfangskonzentrationen $[H_2O_2]_0 = [NO_2^-]_0 = 20$ mM sowie einem pH-Wert von 3,2 und einer Temperatur von 37 °C. Der gefüllte Bereich illustriert das Integral über die Einwirkzeit von $t_1 = 15$ s bis $t_2 = 75$ s.

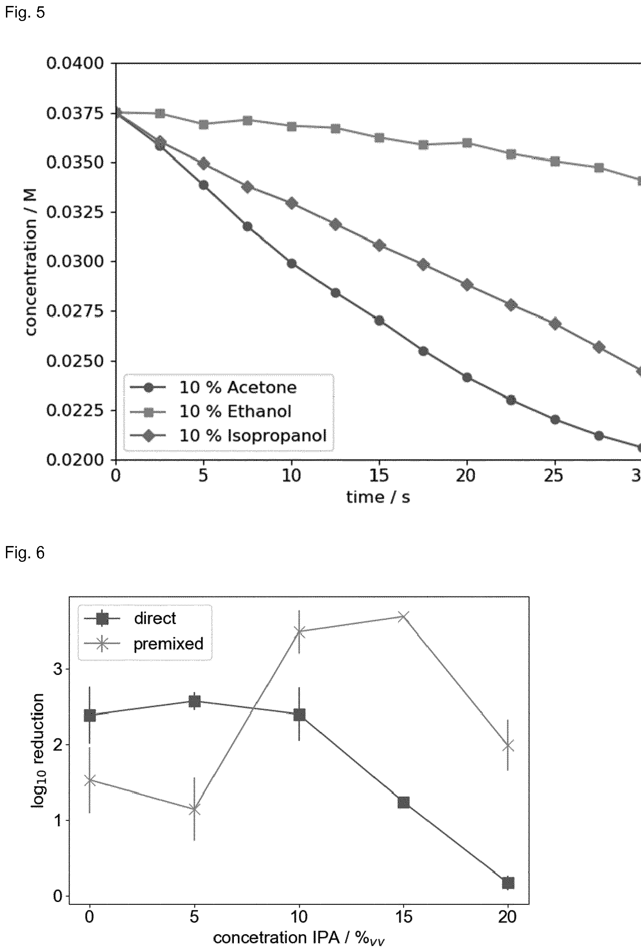

Figur 5 zeigt den Konzentrationsverlauf von Nitrit bei Zugabe von 10 % Ethanol, Aceton oder Isopropanol.

Figur 6 zeigt die Inaktivierung von Sporen von *Bacillus atrophaeus* im Experiment ohne vorherige Mischung der Edukte (direct) sowie mit vorheriger Mischung der Edukte (premixed) (s. Abschnitt "Mikrobiologische Untersuchungen").

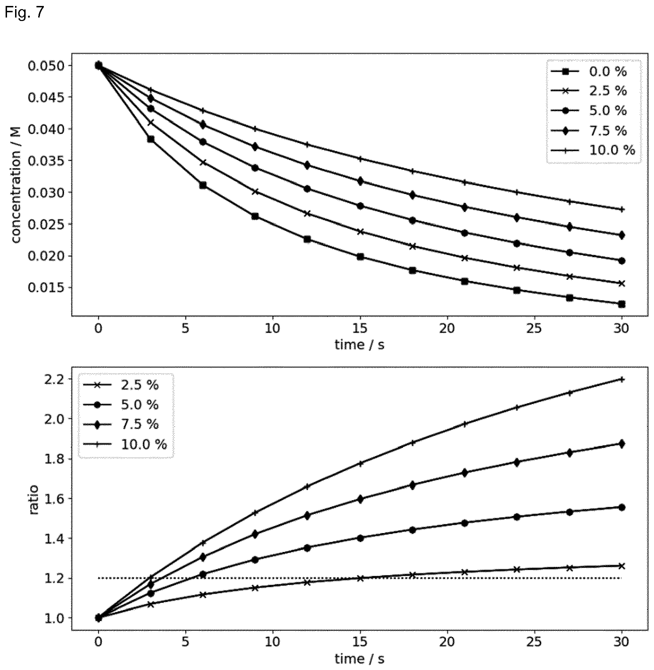

Figur 7 zeigt den zeitlichen Verlauf der Konzentrationen $[H_2O_2]$ und $[NO_2^-]$ bei Anfangskonzentrationen $[H_2O_2]_0 = [NO_2^-]_0 = 50$ mM, einem pH-Wert von 3,2 bei 20 °C (oben) sowie das Verhältnis $n_{t_1}^{min}(x) / n_{t_1}^{min}(0)$ (unten). Die gestrichelte Linie gibt das Verhältnis $n_{t_1}^{min}(x) / n_{t_1}^{min}(0) = 1{,}2$ an.

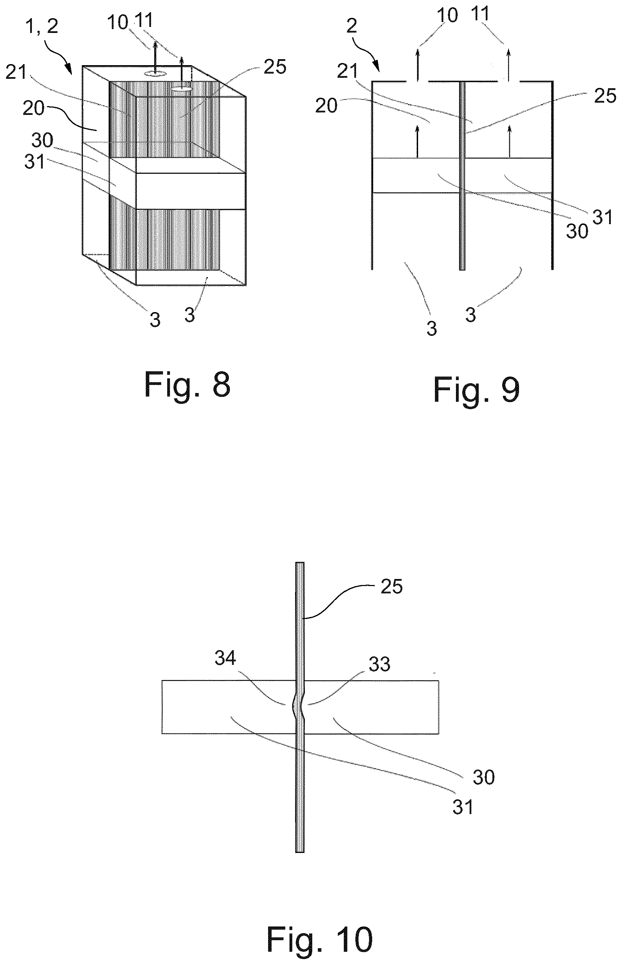

Fig. 8 zeigt eine erfindungsgemäße Vorrichtung in perspektivischer Ansicht,

Fig. 9 zeigt eine Schnittansicht der in Figur 1 dargestellten Vorrichtung,

Fig. 10 zeigt einen Teilbereich der Schnittansicht aus Figur 2,

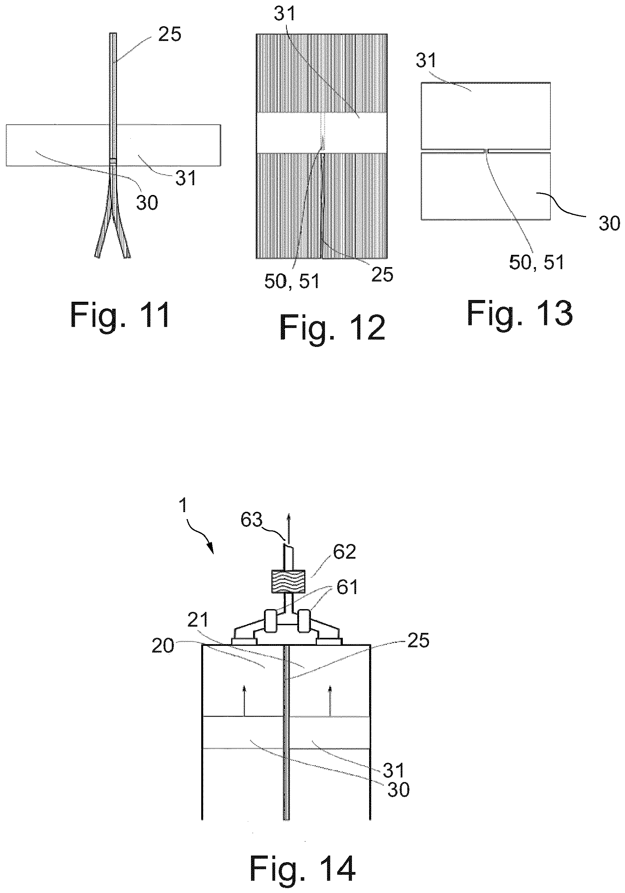

Fig. 11 zeigt einen Teilbereich der Schnittansicht aus Figur 2 bei einem Schneidvorgang,

Fig. 12 zeigt den Schneidvorgang in einer anderen Seitenansicht,

Fig. 13 zeigt die beiden Kolben mit Verbindungselement in Ansicht von oben,

Fig. 14 zeigt einen Teilbereich der erfindungsgemäßen Vorrichtung mit Mischeinheit und erster Pumpe,

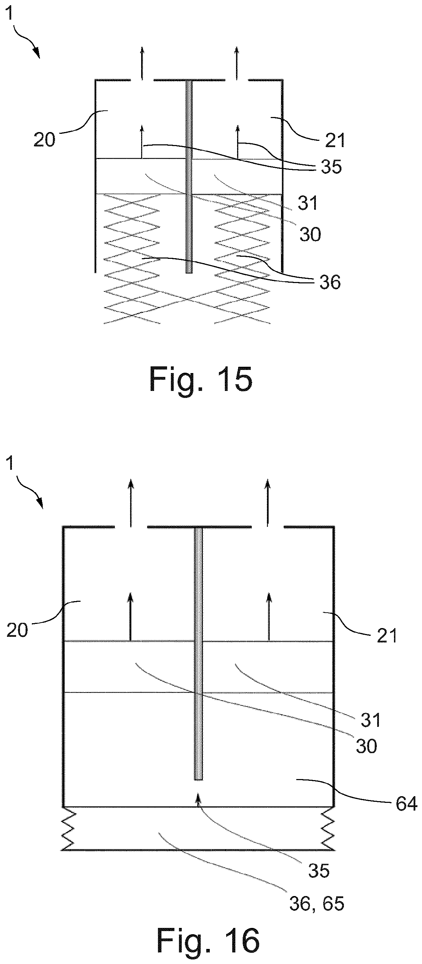

Fig. 15 zeigt die erfindungsgemäße Vorrichtung mit zwei Kraftübertragungselementen,

Fig. 16 zeigt die erfindungsgemäße Vorrichtung mit Druckkammer,

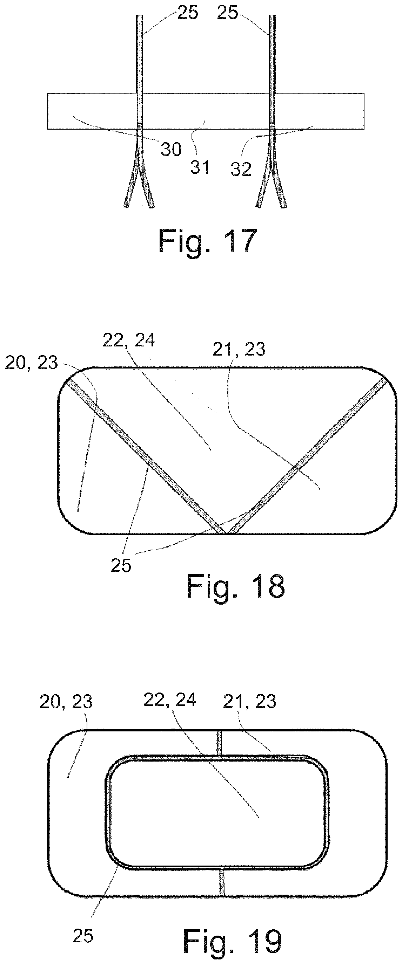

Fig. 17 zeigt drei miteinander verbundene Kolben beim Schneidvorgang,

Fig. 18 zeigt drei Reservoirs mit dreieckigem Querschnitt, und

Fig. 19 zeigt drei Reservoirs mit zentraler Anordnung eines Reservoirs.

### Detaillierte Beschreibung der Figuren 8 bis 19

Eine Kartusche 2 als Teil der erfindungsgemäßen Vorrichtung 1 ist in den Figuren 8 und 9 exemplarisch mit zwei Reservoirs 20,21 dargestellt. In Figur 8 zu erkennen ist eine Kartusche 2, welche über zwei Reservoirs 20,21 verfügt, welche durch eine als Membran ausgeführte Trennwand 25 voneinander getrennt sind. Die Vorrichtung 1 verfügt über zwei Öffnungen 3, in welche die jeweiligen Fluide eingebracht werden können. Die Kolben 30,31 sind in den Reservoirs 20,21 entlang einer Bewegungsrichtung verschiebbar, wobei sie sich lediglich in Vorschubrichtung, also tangential zur Trennwand 25 bewegen lassen. Durch die Bewegung der Kolben 30,31 erfolgt eine Minderung des Volumens eines jeweiligen Reservoirs 20,21, so dass in einem jeweiligen Reservoir 20,21 aufgenommenes Fluid ausgestoßen wird.

Die Bewegung der beiden Kolben 30,31 kann hierbei nicht unabhängig voneinander geschehen. Die Vorrichtung 1 ist so ausgeführt, dass sich die Kolben 31,31 nur synchron bewegen können, sodass sie stets in gleichem Maße einen jeweiligen Volumenstrom 10,11 an Fluid erzeugen. Insbesondere können beide Reservoirs 20,21 die gleiche Größe und beide Kolben 30,31 den gleichen Querschnitt aufweisen, sodass die beiden Volumenströme 10,11 ebenfalls gleich groß sind.

Figur 10 zeigt die Kolben 30,31 und eine als Membran ausgeführte Trennwand 25 der erfindungsgemäßen Vorrichtung 30,31, bei welcher die Kolben 30,31 dadurch indirekt mechanisch gekoppelt sind, dass ein erster Kolben 30 über einen Vorsprung 33 verfügt, und ein zweiter, benachbarter Kolben 31 über eine hinsichtlich Form und Größe komplementär ausgestaltete Aussparung 34 verfügt, so dass der Vorsprung 33 indirekt in die Aussparung 34 eingreift und derart bei Bewegung des einen Kolben 30,31 der andere Kolben 30,31 mitgenommen wird. Die als Membran ausgestaltete Trennwand 25 ist hierbei so wenig biegesteif bzw. flexibel ausgelegt, dass sie eine jeweilige Biegezone 26 im Bereich des Eingriffs des Vorsprungs 33 in die Aussparung 34 ausbilden kann.

Die Figuren 11, 12 und 13 zeigen Kolben 30,31 und Trennwand 25 einer Ausführung der Vorrichtung 1, bei welcher die Kolben 30,31 mittels eines Verbindungselements 50 mechanisch gekoppelt sind, sodass sie nur zusammen verschiebbar sind. Das Verbindungselement 50 ist als eine Klinge 51 ausgestaltet. Insbesondere können hierbei die Kolben 30,31 und das Verbindungselement 50 aus dem gleichen Material bestehen, sodass sich eine einzelne, zusammenhängende Kolben-Einheit ergibt.

In Figur 14 ist dargestellt, wie die Fluid durch eine erste Pumpe 60 entnommen werden kann, welche einen Sog auf die Fluide in den Reservoirs 20,21 ausübt. Hierbei wird eine unkontrollierte Vermischung der beiden Fluide in den Reservoirs 20,21 durch zwei Rückschlagventile 61 vermieden. Die Vermischung der beiden Fluide findet stromabwärts der Ventile 61 statt, im sogenannten Totvolumen, welches von einer Mischeinheit 62 realisiert wird, sich stromaufwärts der ersten Pumpe 60 befindet. Im Gegensatz zur Entnahme der Flüssigkeiten mit zwei einzelnen Pumpen hat diese Ausführung den Vorteil, dass bei Fehlfunktion der ersten Pumpe 60 keine Flüssigkeit abgegeben werden kann, sodass die Fehlfunktion für den Nutzer direkt ersichtlich ist.

Das Gemisch der Fluide kann aus der Düse 63 zur weiteren Verwendung als Flüssigkeit abgegeben, vernebelt oder versprüht werden.

In Figur 15 ist eine Ausführung der Vorrichtung 1 dargestellt, bei welcher der Vorschub der Kolben 30,31 mechanisch realisiert ist, wobei an einen jeweiligen Kolben ein Kraftübertragungselement 36 angeschlossen ist, sodass beide Kolben 30,31 synchron über Einleitung von Kräften durch die Kraftübertragungselemente 36 verschoben werden können.

In Figur 16 ist eine Ausführung der Vorrichtung 1 dargestellt, bei welchem der Vorschub der Kolben 35 durch Wirkung eines Gasdruckes auf die Kolben 30,31 realisiert wird. Hierbei ist den beiden dargestellten Kolben 30,31 eine gemeinsame Druckkammer 64 zugeordnet sowie eine zweite Pumpe 65, die zur Erzeugung eines Überdrucks in der Druckkammer 64 eingerichtet ist, sodass aufgrund des Überdrucks die beiden Kolben 30,31 gleichzeitig bzw. synchron verschoben werden können.

In Figur 17 ist eine Ausführung der Vorrichtung 1 mit drei Kolben 30,31,32 beim Schneidvorgang dargestellt, wobei die Kopplung der Kolben hier exemplarisch dadurch realisiert ist, dass die Kolben 30,31,32 als ein zusammenhängender Kolben ausgeführt sind. Zwischen jeweils zwei der drei Kolben 30,31,32 ist jeweils eine Trennwand 25 angeordnet.

Figur 18 zeigt ein erstes Reservoir 20, zweites Reservoir 21 und drittes Reservoir 22, und dass die Trennwände 25 nicht zwingenderweise parallel zueinander angeordnet sein müssen. Insbesondere kann mindestens ein Reservoir 20,21,22 mindestens ein anderes Reservoir ganz oder teilweise umschließen, wie in Figur 18 dargestellt. Dies ist vorteilhaft, um das Fluid im inneren Reservoir, insbesondere eine $H_2O_2$-haltige Flüssigkeit, vor Lichteinstrahlung und daraus folgender Zersetzung von $H_2O_2$ zu schützen, beispielsweise indem dem Fluid in einem äußeren Reservoir ein entsprechender Farbstoff zugefügt wird, wodurch die Transluzenz des Fluids im äußeren Reservoir und demzufolge die Lichteinstrahlung auf das Fluid im Inneren Reservoir verringert wird.

Figur 19 zeigt eine beispielhafte Anordnung von drei Reservoirs 20,21,22 der Vorrichtung 1, wobei die beiden äußeren Reservoirs 23 das innere Reservoir 24 umhüllen, ebenfalls um die Lichteinstrahlung in die Flüssigkeit im inneren Reservoir 24 zu verringern bzw. zu vermeiden.

Beispiele

*Desinfektionsprozess ohne retardierendes Lösungsmittel*

Die pH-Wert-abhängige Geschwindigkeitskonstante $k=k_0$ kann folgendermaßen berechnet werden:

$$k_0 = k^* \frac{[H_3O^+]^2}{\left(K_{S,H_3O_2^+} + [H_3O^+]\right)\left(K_{S,HNO_2} + [H_3O^+]\right)} \qquad (40)$$

mit

$$k^* = 3{,}56 \cdot 10^{14} \exp\left(-\frac{E_A}{RT}\right) M^{-1} s^{-1} \qquad (50)$$

$$K_{S,HNO_2} = 5{,}13 \times 10^{-4} \qquad\qquad (60)$$

$$K_{S,H_3O_2^+} = 2 \times 10^{-2} \qquad\qquad (70)$$

und der einheitenlosen Größe

$$[H_3O^+] = 10^{-pH} \qquad\qquad (80)$$

mit der effektiven Aktivierungsenergie $E_A$=70 kJ/mol und der Temperatur T.

Exemplarisch erhält man durch Lösen der sich aus (2) ergebenden Differentialgleichungen für die Konzentrationen [$H_2O_2$] und [$NO_2^-$] bei gleichen Startkonzentrationen zum Zeitpunkt $t_0$ von [$H_2O_2$]$_0$ = [$NO_2^-$]$_0$=20 mM sowie einem pH-Wert von 3,2 und einer Temperatur von 37 °C die in Figur 1 oben gezeigten Konzentrationsverläufe. In Figur 1 unten ist die Reaktionsrate nach Gleichung (3) angegeben. Der gefüllte Bereich in Figur 1 unten verdeutlicht das Integral (1) mit $t_0$ = 15 s und $t_1$ = 75 s, wobei sich in diesem Fall ein Wirksamkeitsparameter von W(15 s bis 75 s) = 3,8 mM ergibt. Wie aus Figur 1 unten leicht ersichtlich, ist das Mittel während des Verteilungsschritts wirksamer als während der eigentlichen Einwirkzeit: Berechnet man das Integral (1) während der Verarbeitungszeit, also mit $t_0$ = 0 s und $t_1$ = 15 s, ergibt sich ein Wirksamkeitsparameter von W(0 s bis 15 s) = 14,7 mM. Ein Großteil des Wirkpotentials wird somit nicht ausgenutzt. Die Dauer des Verteilungsschrittes hängt vom jeweiligen Prozess ab und kann nicht frei gewählt bzw. beliebig verkürzt werden. So ist beispielsweise bei der hygienischen Händedesinfektion eine Verteilzeit von 30 s üblich.

*Durchgeführte Experimente*

*Einfluss von Isopropanol auf die Geschwindigkeitskonstante*

In Figur 2 ist der Einfluss von zu den Wirklösungen zugefügtem Isopropanol (IPA) auf die Geschwindigkeitskonstante (40) bei einer Temperatur von 20 °C dargestellt. Die Messung der Reaktionskonstante erfolgte hierbei durch UV-Spektroskopie, wobei die Abnahme der $NO_2^-$-Konzentration zur Bestimmung der Reaktionsrate quantifiziert wurde. Wie aus Figur 2 ersichtlich, kann die Reaktion (2) durch Hinzufügen von Isopropanol effektiv verlangsamt werden. Der Einfluss der Isopropanolkonzentration auf die Reaktionsrate der Reaktion (2) kann berücksichtigt werden, indem die Geschwindigkeitskonstante $k_0$ mit einem von der Isopropanolkonzentration IPA (angegeben in Volumenprozent) abhängigen Faktor gemäß

$$k = k_0 \times \exp(-0{,}129 \times \mathrm{IPA}) \qquad\qquad (90)$$

multipliziert wird.

*Zeitliche Änderung der Isopropanolkonzentration auf Oberflächen*

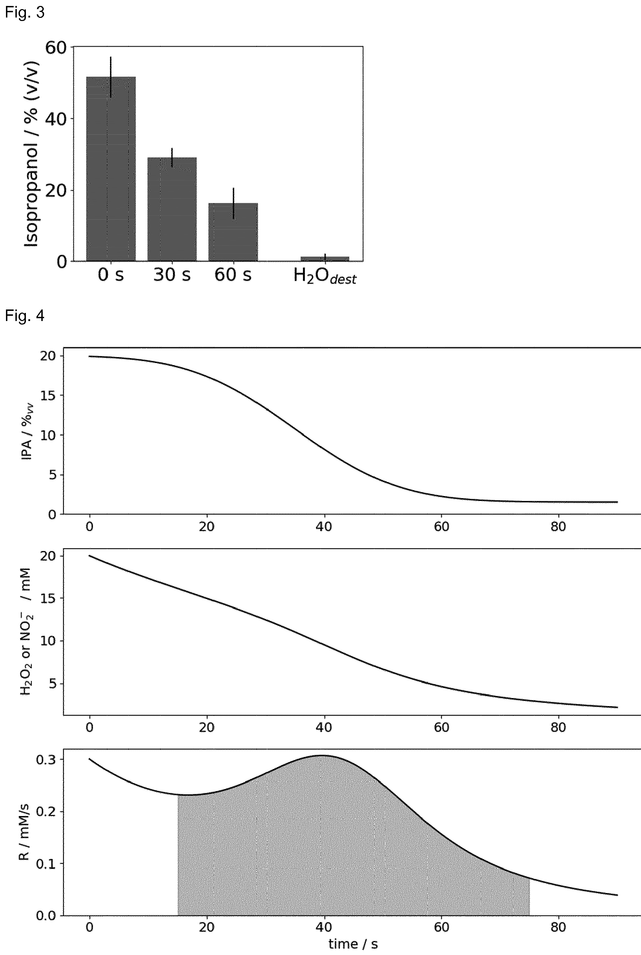

Wird eine Lösung aus Wasser und einem Lösungsmittel mit höherem Dampfdruck als Wasser auf eine Oberfläche aufgebracht, verdampft das Lösungsmittel schneller, wodurch dessen Anteil in der Lösung sinkt. Dazu wurde folgendes Experiment durchgeführt:
Eine Metallplatte mit einer Fläche von 567 cm$^2$ wurde auf eine Temperatur von $(37 \pm 2)$ °C erhitzt. 3 mL einer Isopropanol-Lösung wurde auf der Platte verteilt. Nach einer Wartezeit von 30 s bzw. 60 s wurde die auf der Oberfläche verbliebene Flüssigkeit in einem Gefäß gesammelt und die Dichte der Flüssigkeit bestimmt. Dazu wurde das Gewicht von 100 µL der aufgesammelten Flüssigkeit gemessen. Aus den in Chu, Kwang-Yu, and A. Ralph Thompson. Journal of chemical and engineering data 7.3 (1962): 358-360 angegebenen Werten zur Konzentrationsabhängigkeit der Dichte von Isopropanollösungen wurde die Isopropanolkonzentration der aufgesammelten Flüssigkeit ermittelt. Des Weiteren wurde zur Überprüfung des Verfahrens die Dichte der Isopropanollösung vor Aufbringen auf die Metallplatte (in Figur 3 bezeichnet mit "0 s") sowie von destilliertem Wasser ($H_2O$ *dest*) bestimmt. Die gewählte Temperatur und Fläche ist insbesondere als Modell für die Händedesinfektion relevant. Bei dieser wird die Wirklösung ebenfalls durch Körperwärme und Reibungswärme bei der vorschriftsmäßigen Durchführung der Händedesinfektion erwärmt. Zudem tritt bei der Händedesinfektion, aufgrund der Oberflächenbeschaffenheit der Haut, ein noch größeres Oberflächen- zu Volumenverhältnis der verteilten Wirklösung auf.

*Zeitliche Änderung der Reaktionsrate (3) auf Oberflächen*

Die Konzentrationsabhängigkeit (90) zusammen mit der in Figur 3 dargestellten zeitlichen Änderung der Isopropanol-Konzentration kann ausgenutzt werden, um das Ablaufen der Reaktion (2) zu retardieren. In Figur 4 (oben) ist der zeitliche Verlauf einer angenommenen Isopropanol-Konzentration bei einer Oberflächendesinfektion mit einer Lösung aus $H_2O_2$ und $NO_2^-$ dargestellt. Figur 4 (mitte) zeigt die aus Gleichungen (1) und (90) resultierenden Konzentrationen von $[H_2O_2]$ und $[NO_2^-]$, wobei die Anfangskonzentrationen $[H_2O_2]_0 = [NO_2^-]_0 = 20$ mM, der pH-Wert 3,2 und die Temperatur 37 °C betragen. Es wurden somit, mit Ausnahme der Isopropanol-Konzentration, die gleichen Bedingungen wie bei der in Figur 1 dargestellten Berechnung gewählt. Jedoch beträgt der Wirksamkeitsparameter bei einem Start der Einwirkzeit bei $t_1 = 15$ s und Ende bei $t_2 = 75$ s hier aufgrund der retardierten Reaktion $W_{IPA}(15$ s bis $45$ s$) = 13,1$ mM. Dies ist um 9,3 mM höher verglichen mit dem ohne IPA-Einsatz erzielten Wert von $W(15$ s bis $45$ s$) = 3,8$ mM. Hierdurch ist gezeigt, dass durch Hinzufügen eines Lösungsmittels, welches die Reaktionsrate der Reaktion (2) vermindert, ein wirksameres Desinfektionsmittel entsteht.

In Figur 5 ist der Einfluss von unterschiedlichen Lösungsmitteln auf die Reaktionsgeschwindigkeit der Reaktion 2 dargestellt. Die Messungen erfolgten mittels UV-Spektroskopie mit einer Absorptionslänge von 1 cm und bei einer Wellenlänge von 332 nm.

*Mikrobiologische Untersuchungen*

Um die retardierte mikrobiologische Wirkung bei Verwenden einer Stopplösung nachzuweisen, wurde die Wirkung der Wirklösung auf Sporen der Art *Bacillus atrophaeus* in zwei Experimenten untersucht.

Im ersten Experiment wurden 10 μL einer Sporenlösung (enthaltend Sporen des Bakteriums der Art *Bacillus atrophaeus*) in einem Reaktionsgefäß vorgelegt. Anschließend wurde 495 μL einer 50 mM $NaNO_2$-Lösung hinzugefügt, im Anschluss wurden 495 μL einer 50 mM $H_2O_2$-Lösung zum Erhalt einer Wirklösung hinzugefügt. Hierbei enthielten sowohl die $NaNO_2$-Lösung als auch die $H_2O_2$-Lösung jeweils die gleiche Konzentration von Isopropanol, ausgewählt aus 0%, 5%, 10%, 15% oder 20%, wobei sich die Prozentangaben auf Volumenprozent beziehen. Zudem wurde die $H_2O_2$-Lösung mittels 25 mM $H_3PO_4$ angesäuert. Die Reaktion wurde nach einer Einwirkzeit von 60 s durch Verdünnung in einer Neutralisationslösung gestoppt und anschließend auf Agar ausplattiert. Nach einer Inkubationszeit von 24 h wurden die Koloniebildenden Einheiten auf der jeweiligen Agarplatte quantifiziert.

Die Ergebnisse dieses Versuches sind in Figur 6 (Messreihe "direct") dargestellt. Die angegebene "log10 reduction" ist hierbei der negative dekadische Logarithmus der ermittelten Konzentration Koloniebildender Einheiten nach Anwendung der jeweiligen Wirklösung im Verhältnis zur ermittelten Bakterienkonzentration in einer Negativkontrolle. Wie aus den Daten ersichtlich, führt die Zugabe von Isopropanol zu einer Verschlechterung der Wirkung der jeweiligen Wirklösung. Im Lichte der vorherigen Untersuchungen ist klar, dass die Verschlechterung der Wirkung auf eine Senkung der Reaktionsrate, beispielsweise entsprechend Gleichung (90) zurückzuführen ist.

Im zweiten Experiment wurde analog zum ersten Experiment 10 μL einer Sporenlösung (*B. atrophaeus*) vorgelegt. In einem separaten Reaktionsgefäß wurde 1 mL einer 75 mM $NaNO_2$-Lösung vorgelegt und mit 1 mL einer 75 mM $H_2O_2$-Lösung zum Erhalt einer Wirklösung zur Reaktion gebracht. Nach 15 s Reaktionszeit wurde 990 μL dieser Wirklösung zur Sporenlösung gegeben. Analog zum ersten Experiment enthielten sowohl die $NaNO_2$-Lösung als auch die $H_2O_2$-Lösung jeweils die gleiche Konzentration von Isopropanol, ausgewählt aus 0%, 5%, 10%, 15% oder 20%. Zudem wurde die $H_2O_2$-Lösung mittels 37,5 mM $H_3PO_4$ angesäuert. Die im Vergleich zum ersten Experiment höheren Konzentrationen der Edukte wurden hierbei gewählt, um den Verlust dieser Edukte während der 15 s Reaktionszeit annähernd auszugleichen. Analog zum ersten Experiment wurde die Lösung nach 60 s Einwirkzeit in Neutralisationslösung verdünnt und ausplattiert.

Die Ergebnisse dieses Versuches sind in Figur 6 (Messreihe "premixed") dargestellt. Wie hieraus ersichtlich, führt die Zugabe von Isopropanol in diesem Experiment für Isopropanolkonzentrationen von bis zu 15 % zu einer Verbesserung der sporiziden Wirkung - der Trend läuft also Entgegen der Beobachtung im ersten Experiment. Dies ist jedoch auch hier darauf zurückzuführen, dass das Isopropanol hier als Stopplösung fungiert. Dies hat zur Folge, dass die Reaktion in den 15 s Reaktionszeit langsamer abläuft, sodass während der Einwirkzeit noch mehr Edukte zur Verfügung stehen. Bei einer Isopropanol-Konzentration von 20 % wird die Reaktion in diesem Experiment bereits so stark verlangsamt, dass während der Einwirkzeit weniger Edukte (verglichen mit dem 15 %-Experiment) umgesetzt werden und die Wirkung somit schlechter ist.

*Beispiel zur Bestimmung der minimal einsetzbaren Lösunqsmittelkonzentration*

*Definitionen*

- Die Gesamtprozessdauer ist die Verteilzeit + Trocknungszeit.
  Die Verteilzeit endet zum Zeitpunkt $t_1$.
- Trocknungszeit = Zeit bis benetzte Oberfläche komplett getrocknet ist, endet zum Zeitpunkt $t_2$.
- $c_{t_1}^{min}(x) = min(c_{H_2O_2}(x, t=t_1), c_{NO_2^-}(x, t=t_1))$, wobei x die Konzentration des Stoppagens in Volumenprozent bezogen auf das Volumen der Wirklösung zum Zeitpunkt $t=t_0$ ist.
  Die Funktion min($a, b$) ist gleich a wenn a<b, b wenn b≤a.

$c_{t_1}^{min}(0)$ bezieht sich auf eine Wirklösung ohne Stoppagens.

$c_{t_1}^{min}$ entspricht der maximal erzielbaren Wirksamkeit $W = \int_{t_1}^{\infty} k \cdot [H_2O_2] \cdot [NO_2^-]\, dt$, wobei k die Geschwindigkeitskonstante der Reaktion zwischen $H_2O_2$ und $NO_2^-$ bezeichnet Vorteilhaft ist, wenn die Gesamtprozessdauer möglichst kurz ist. Vorteilhaft ist außerdem eine möglichst große Wirksamkeit während der Trocknungszeit. Eine Verkürzung der Trocknungszeit ist durch Zugabe eines Alkohols mit einer niedrigeren Siedetemperatur als Wasser stets gegeben.

Zudem wird die Wirksamkeit durch Zugabe von Alkohol in der Trocknungszeit erhöht. Bei der Auslegung der Alkoholkonzentration sind folgende Punkte zu berücksichtigen:

a) Minimaler Alkoholzusatz:
Die Alkoholkonzentration muss so gewählt werden, dass die Bedingung

$$\frac{c_{t_1}^{min}(x)}{c_{t_1}^{min}(0)} > 1{,}2 \quad (100),$$

erfüllt ist.

b) Maximaler Alkoholzusatz:
Eine zu hohe Alkoholkonzentration kann zu ungewollten Veränderungen der behandelten Oberflächen, bzw. im Falle der Anwendung auf der Haut zu Hautreizungen führen, daher ist die Alkoholkonzentration so niedrig wie möglich zu wählen. Insbesondere soll die Alkoholkonzentration kleiner 90 %, insbesondere kleiner 60 %, insbesondere kleiner 40 %.

In Figur 7 (oben) ist exemplarisch der Konzentrationsverlauf für $NO_2^-$ und $H_2O_2$ für Zumischungen von 0, 2.5, 5.0, 7.5, und 10.0 % Isopropanol dargestellt. In Figur 7 (unten) sind die Verhältnisse $n_{t_1}^{min}(x)/n_{t_1}^{min}(0)$ dargestellt. Zudem gibt die gestrichelte Linie die geforderte 20 %ige Verbesserung an, sodass sich nach Gleichung (X) der Erfindungsgemäß zu wählende Bereich ablesen lässt.

*Bestimmung der minimal einsetzbaren Lösungsmittelkonzentration*

Benötigt sei ein Desinfektionsmittel, welches eine Verteilzeit von mindestens 15 s erlaubt, einen pH-Wert von 3.2 aufweist und wobei als retardierendes Lösungsmittel Isopropanol zum Einsatz kommt. Folgende Schritte sind dazu durchzuführen:

1. Die Konzentration von $H_2O_2$ und $NO_2^-$ sind bei gegebenen pH-Wert nach Mischen der Komponenten für verschiedene Isopropanolkonzentrationen zeitaufgelöst zu messen. Dies kann beispielsweise durch UV-Spektroskopie, wie PCT/EP2019/062897 und oben angegeben erfolgen (vgl. Figur 7 oben).

2. Für die gewählten Isopropanolkonzentrationen x ist das Verhältnis $n_{t_1}^{min}(x)/n_{t_1}^{min}(0)$ zu bestimmen (vgl. Figur 7 unten) und die Bedingung $n_{t_1}^{min}(x)/n_{t_1}^{min}(0) > 1{,}2$ zu überprüfen. In diesem Beispiel ergibt sich, dass eine Isopropanolkonzentration von 2,5 % die minimal einsetzbare Lösungsmittelkonzentration ist.

Für andere Lösungsmittel und pH-Werte ist analog vorzugehen.

Literaturverzeichnis:

Zhu, Ling, Christopher Gunn, and Joseph S. Beckman. "Bactericidal activity of peroxynitrite." Archives of biochemistry and biophysics 298.2 (1992): 452-457.

Chu, Kwang-Yu, and A. Ralph Thompson. "Densities and Refractive Indices of Alcohol-Water Solutions of n-Propyl, Isopropyl, and Methyl Alcohols." Journal of chemical and engineering data 7.3 (1962): 358-360.

**Patentansprüche**

1.  Ein Verfahren zur Desinfektion von Oberflächen umfassend die Bereitstellung einer Wirklösung umfassend die Edukte $H_2O_2$ und $NO_2^-$,
    **dadurch charakterisiert, dass**
    die Wirklösung mindestens ein Stoppagens zur Verminderung der Reaktionsgeschwindigkeit von $H_2O_2$ und $NO_2^-$ umfasst, wobei das Stoppagens ein Lösungsmittel ist, welches eine Siedetemperatur unter 100 °C aufweist.

2.  Das Verfahren nach Anspruch 1, wobei das Stoppagens ausgewählt ist aus einem Alkohol, einem Keton und einem Ester, insbesondere Methanol, Ethanol, Isopropanol, Aceton, Ethylacetat und n-Propanol, weiter insbesondere Ethanol, Isopropanol und Aceton.

3.  Das Verfahren nach einem der Ansprüche 1 oder 2, wobei die Wirklösung durch das Vermischen der Edukte $H_2O_2$ und $NO_2^-$ und dem Stoppagens zum Zeitpunkt $t_0$ erhalten wird.

4.  Das Verfahren nach einem der vorhergehenden Ansprüche, wobei die Wirklösung auf einer zu desinfizierenden Oberfläche bis zur vollständigen Benetzung zum Zeitpunkt $t_1$ verteilt wird.

5.  Das Verfahren nach einem der vorhergehenden Ansprüche, wobei der Zeitraum zwischen $t_0$ und $t_1$ mindestens 5 Sekunden, insbesondere mindestens 10 Sekunden, weiter insbesondere mindestens 15 Sekunden beträgt.

6.  Das Verfahren nach einem der vorhergehenden Ansprüche, wobei die Wirklösung bis zum Zeitpunkt $t_2$ zum Erhalt einer desinfizierten Oberfläche einwirkt.

7.  Das Verfahren nach einem der vorhergehenden Ansprüche, wobei die minimale Konzentration des Stoppagens in der Wirklösung zum Zeitpunkt $t_0$ mindestens 2.5 % (v/v) und/oder die maximale Konzentration des Stoppagens in der Wirklösung < 90 % (v/v), insbesondere < 60 % (v/v), weiter insbesondere < 40 % (v/v) beträgt.

8.  Das Verfahren nach einem der vorhergehenden Ansprüche, wobei der pH-Wert der Wirklösung zum Zeitpunkt $t_0$ zwischen 1. und 7 liegt, insbesondere zwischen 2 und 6, insbesondere zwischen 3 und 5 liegt.

9.  Das Verfahren nach einem der vorhergehenden Ansprüche, wobei die Ausgangskonzentration $[H_2O_2]_0$ zum Zeitpunkt $t_0$ zwischen 1 mM und 1000 mM liegt, insbesondere zwischen 10 mM und 500 mM, insbesondere zwischen 15 und 300 mM liegt.

10. Das Verfahren nach einem der vorhergehenden Ansprüche, wobei die Ausgangskonzentration $[NO_2^-]_0$ zum Zeitpunkt $t_0$ zwischen 1 mM und 1000 mM liegt, insbesondere zwischen 10 mM und 500 mM, insbesondere zwischen 15 und 300 mM liegt.

11. Vorrichtung 1 zur simultanen Abgabe von zumindest zwei Volumenströmen 10,11 von $H_2O_2$ und $NO_2^-$ Lösungen, insbesondere von zumindest zwei Volumenströmen 10,11 gleicher Größe, umfassend zumindest zwei Reservoirs 20,21 zur Aufnahme von $H_2O_2$ und zur Aufnahme von $NO_2^-$, und in einem jeweiligen Reservoir 20,21 angeordnet einen verschiebbaren Kolben 30,31 zur Förderung eines Fluids aus dem jeweiligen Reservoir, wobei die Kolben 30,31 über eine Kraftübertragungseinrichtung derart miteinander gekoppelt sind, dass sie synchron parallel zueinander verschiebbar sind, so dass die Fluide aus den Reservoirs 20,21 zur selben Zeit, insbesondere mit gleichen Volumenströmen 10,11, ausbringbar sind.

12. Vorrichtung 1 zur simultanen Abgabe von zumindest zwei Volumenströmen 10,11 von $H_2O_2$ und $NO_2^-$ nach Anspruch 1, **dadurch gekennzeichnet, dass** die beiden Reservoirs 20,21 durch zumindest eine gemeinsame Trennwand 25 voneinander abgeteilt sind, wobei diese Trennwand 25 eine geringere Biegefestigkeit aufweist als die an der Trennwand 25 gleitenden Seiten der Kolben 30,31, und wobei ein erster Kolben 30 einen in Richtung auf einen zweiten Kolben 31 vorstehenden Vorsprung 33 aufweist und der zweite Kolben 31 eine im Wesentlichen hinsichtlich Form und Größe des Vorsprungs 33 komplementäre Aussparung 34 aufweist, sodass bei Verschiebung eines Kolben der jeweils andere Kolben unter Verformung der Trennwand 25 über einen mittelbaren mechanischen Eingriff des Vorsprungs 33 in der Aussparung 34 mitgenommen wird.

13. Vorrichtung 1 zur simultanen Abgabe von zumindest zwei Volumenströmen 10,11 von $H_2O_2$ und $NO_2^-$ nach Anspruch 12, **dadurch gekennzeichnet, dass** die Reservoirs 20,21 durch zumindest eine gemeinsame Trennwand 25 voneinander abgeteilt sind, wobei die Kolben 30,31 über wenigstens ein Verbindungselement 50 verbunden sind, welches dazu eingerichtet ist, bei einer Verschiebung der Kolben 30,31 die dazwischen befindliche Trennwand 25 zumindest abschnittsweise zu zerschneiden.

14. Vorrichtung 1 zur simultanen Abgabe von zumindest zwei Volumenströmen von $H_2O_2$ und $NO_2^-$ nach einem der vorhergehenden Ansprüche, **dadurch gekennzeichnet, dass** die Vorrichtung drei Reservoirs 20,21,22 aufweist, wobei in dem dritten Reservoir 22 ein verschiebbarer dritter Kolben 32 zur Förderung eines Fluids aus dem dritten Reservoir 22 angeordnet ist, wobei die drei Kolben 30,31,32 über eine Kraftübertragungseinrichtung derart miteinander gekoppelt sind, dass sie synchron parallel zueinander verschiebbar sind, so dass die Fluide aus den Reservoirs 20,21,22 mit gleichen Volumenströmen ausbringbar sind.

15. Vorrichtung zur simultanen Abgabe von zumindest zwei Volumenströmen von $H_2O_2$ und $NO_2^-$ nach einem der vorherigen Ansprüche, **dadurch gekennzeichnet, dass** zumindest ein erstes Reservoir 20 an wenigstens zwei Seiten von wenigstens einem weiteren Reservoir 21,22 benachbart ist, wobei in dem weiteren Reservoir 21,21 das Fluid eine geringere Transluzenz aufweist als das Fluid im ersten Reservoir 20 zwecks Verringerung der Lichteinstrahlung in das Fluid im ersten Reservoir 20.

Fig. 1

Fig. 2

Fig. 3

Fig. 4

Fig. 5

Fig. 6

Fig. 7

Fig. 8

Fig. 9

Fig. 10

25 31 30

**Fig. 11**

31 50, 51 25

**Fig. 12**

31 30 50, 51

**Fig. 13**

1 63 62 61 21 20 25 31 30

**Fig. 14**

Fig. 15

Fig. 16

Fig. 17

Fig. 18

Fig. 19

Europäisches Patentamt
European Patent Office
Office européen des brevets

# EUROPÄISCHER RECHERCHENBERICHT

Nummer der Anmeldung

EP 19 19 5459

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | EP 3 189 857 A1 (KITANO KATSUHISA [JP]; TANI ATSUSHI [JP] ET AL.) 12. Juli 2017 (2017-07-12) * Ansprüche; Abbildungen * * Absätze [0050], [0055], [0059], [0067] * ----- | 1-10 | INV. A61L2/18 B01F15/04 B65D25/08 B65D81/32 ADD. A61L2/14 A01N59/00 A23L3/358 |
| Y | US 2017/142962 A1 (TSAI TSUNG-CHAN [US] ET AL) 25. Mai 2017 (2017-05-25) * Absätze [0048], [0050], [0055] * ----- | 1-10 | |
| Y | W. HEASELGRAVE ET AL: "Acidified nitrite enhances hydrogen peroxide disinfection of Acanthamoeba, bacteria and fungi", JOURNAL OF ANTIMICROBIAL CHEMOTHERAPY., Bd. 65, Nr. 6, 23. März 2010 (2010-03-23), Seiten 1207-1214, XP055547681, GB ISSN: 0305-7453, DOI: 10.1093/jac/dkq075 * Abbildungen; Tabelle 1 * * Seite 1208 * ----- | 1-10 | |

RECHERCHIERTE SACHGEBIETE (IPC)

A61L
A01N
A61P
A23L
B01F
B65D

~~Der vorliegende Recherchenbericht wurde für alle Patentans~~prüche erstellt

| Recherchenort | Abschlußdatum der Recherche | Prüfer |
|---|---|---|
| München | 2. März 2020 | Kleiminger, Lisa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

European
Patent Office

Office européen
des brevets

Nummer der Anmeldung

EP 19 19 5459

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-10

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

EP 19 19 5459

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-10

   Zugabe eines retardierendes Stoppagens
   ---

2. Ansprüche: 11-15

   Verbesserte Vorrichtung zur simultanen Mischung von Edukten
   ---

**ANHANG ZUM EUROPÄISCHEN RECHERCHENBERICHT
ÜBER DIE EUROPÄISCHE PATENTANMELDUNG NR.**

EP 19 19 5459

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-03-2020

| Im Recherchenbericht angeführtes Patentdokument | | Datum der Veröffentlichung | Mitglied(er) der Patentfamilie | | | Datum der Veröffentlichung |
|---|---|---|---|---|---|---|
| EP 3189857 | A1 | 12-07-2017 | EP | 3189857 | A1 | 12-07-2017 |
| | | | JP | 6087029 | B2 | 01-03-2017 |
| | | | JP | WO2016035342 | A1 | 27-04-2017 |
| | | | US | 2017172149 | A1 | 22-06-2017 |
| | | | WO | 2016035342 | A1 | 10-03-2016 |
| US 2017142962 | A1 | 25-05-2017 | CA | 3006857 | A1 | 01-06-2017 |
| | | | EP | 3379929 | A1 | 03-10-2018 |
| | | | US | 2017142962 | A1 | 25-05-2017 |
| | | | WO | 2017091534 | A1 | 01-06-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2019062897 W

**In der Beschreibung aufgeführte Nicht-Patentliteratur**

- **CHU ; KWANG-YU ; A. RALPH THOMPSON.** *Journal of chemical and engineering data,* 1962, vol. 7.3, 358-360
- **ZHU ; LING ; CHRISTOPHER GUNN ; JOSEPH S. BECKMAN.** Bactericidal activity of peroxynitrite. *Archives of biochemistry and biophysics,* 1992, vol. 298.2, 452-457
- **CHU ; KWANG-YU ; A. RALPH THOMPSON.** Densities and Refractive Indices of Alcohol-Water Solutions of n-Propyl, Isopropyl, and Methyl Alcohols. *Journal of chemical and engineering data,* 1962, vol. 7.3, 358-360